# EUROPÄISCHE PATENTSCHRIFT

(11) **EP 1 355 619 B1**
(45) Veröffentlichungstag und Bekanntmachung des Hinweises auf die Patenterteilung: **06.12.2006**
(21) Anmeldenummer: 01978353.9
(22) Anmeldetag: 10.09.2001
(51) Int. Cl.: A61K 8/41, A61Q 5/10, C07C 215/74, C07C 217/80, C07C 209/52, C07C 255/58, C07C 225/22, C07C 323/31, C07D 317/58

(54) **(1,1'-BIPHENYL)-2,4-DIAMIN-DERIVATE ENTHALTENDE FÄRBEMITTEL FÜR KERATINFASERN SOWIE NEUE (1,1'-BIPHENYL)-2,4-DIAMIN-DERIVATE**
COLOURING AGENTS FOR KERATIN FIBRES CONTAINING (1.1'-BIPHENYL)-2.4-DIAMINE DERIVATIVES IN ADDITION TO NOVEL (1.1'-BIPHENYL)-2.4-DIAMINE-DERIVATIVES
AGENTS COLORANTS DESTINES A DES FIBRES KERATINIQUES CONTENANT DES DERIVES (1,1'-BIPHENYL)-2,4-DIAMINE, ET NOUVEAUX DERIVES (1,1'-BIPHENYL)-2,4-DIAMINE

(30) Priorität: 02.02.2001 DE 10104770
(43) Veröffentlichungstag der Anmeldung: 29.10.2003
(73) Patentinhaber: Wella Aktiengesellschaft, 64274 Darmstadt (DE)
(72) Erfinder: CHASSOT, Laurent, CH-1724 Praroman (CH); BRAUN, Hans-Jürgen, CH-3182 Überstorf (CH)
(86) Internationale Anmeldenummer: PCT/EP2001/010409
(87) Internationale Veröffentlichungsnummer: WO 2002/062307

(56) Entgegenhaltungen:
- WO-A-99/59527
- DE-A- 2 232 095
- GB-A- 1 425 064
- US-A- 3 619 399
- S. ARAVAMUTHAN ET AL.: "electrochemical reduction of 2,3'-dinitrobenzidine in buffered aqueous methanol" JOURNAL OF APPLIED ELECTROCHEMISTRY, Bd. 19, Nr. 6, 1989, Seiten 897-900, XP001073817

## Beschreibung

Die vorliegende Erfindung betrifft Mittel zur oxidativen Färbung von Keratinfasem, insbesondere menschlichen Haaren, auf der Basis einer Entwicklersubstanz/Kupplersubstanz-Kombination, welche als Kupplersubstanz [1,1'-Biphenyl]-2,4-diamin-Derivate enthalten, sowie neue [1,1'-Biphenyl]-2,4-diamin-Derivate.

Auf dem Gebiet der Färbung von Keratinfasem, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol,4,5-Diamino-1-(2'-hydroxyethyl)pyrazol und 1,4-Diaminobenzol eingesetzt, während als Kupplersubstanzen beispielsweise Resorcin, 2-Methylresorcin, 1-Naphthol, 3-Aminophenol, m-Phenylendiamin, 2-Amino-4-(2'-hydroxyethyl)amino-anisol, 1,3-Diamino-4-(2'-hydroxyethoxy)benzol und 2,4-Diamino-5-fluor-toluol zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.

Es wurde bereits versucht, die Eigenschaften von m-Phenyldiaminen durch die Einführung von Substituenten zu verbessern. In diesem Zusammenhang sei auf die DE-OS 30 28 131 verwiesen, in der unter anderem auch Färbemittel beschrieben werden, welche als Kupplersubstanzen spezielle, in 4-Stellung alkylsubstituierte m-Phenylendiamine enthalten.

Mit den derzeit bekannten Färbemitteln ist es jedoch nicht möglich, die an ein Färbemittel gestellten Anforderungen in allen Punkten zu erfüllen.
Es bestand daher weiterhin ein Bedürfnis nach neuen Kupplersubstanzen, welche die vorgenannten Anforderung in besonders hohem Maße erfüllen.

Es wurde nun gefunden, dass bei Verwendung von [1,1'-Biphenyl]-2,4-diamin-Derivaten der allgemeinen Formel (I) neben Naturtönen und purpurnen oder violetten Farbtönen auch intensive, stabile blaue Farbnuancen erhalten werden.

Gegenstand der vorliegende Erfindung ist daher ein Mittel zur oxidativen Färbung von Keratinfasern, wie zum Beispiel Wolle, Pelzen, Federn oder Haaren, insbesondere menschlichen Haaren, auf der Basis einer Entwicklersubstanz-Kupplersubstanz-Kombination, welches dadurch gekennzeichnet ist, dass es als Kupplersubstanz mindestens ein [1,1'-Biphenyl]-2,4-diamin-Derivat der Formel (I) oder dessen physiologisch verträgliches Salz enthält, 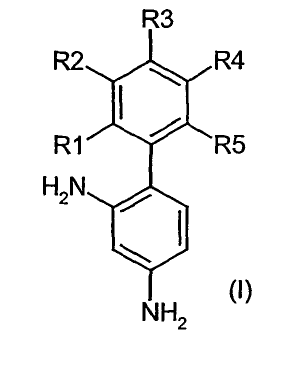 worin
**R1,R2,R3,R4,R5** gleich oder verschieden sein können und unabhängig voneinander Wasserstoff, ein Halogenatom (Fluor, Chlor, Brom, Jod), eine Cyanogruppe, eine Hydroxygruppe, eine C₁-C₄-Alkoxygruppe, eine C₁-C₄-Hydroxyalkoxygruppe, eine C₁-C₆-Alkylgruppe, eine C₁-C₄-Alkylthioethergruppe, eine Mercaptogruppe, eine Nitrogruppe, eine Aminogruppe (NH2), eine C₁-C₄-Monoalkylaminogruppe, eine Di(C₁-C₄-)-alkylaminogruppe, eine Trifluormethangruppe, eine -C(O)H-Gruppe, eine -C(O)CH₃-Gruppe, eine -C(O)CF₃-Gruppe, eine -Si(CH₃)₃-Gruppe, eine C₁-C₄-Hydroxyalkylgruppe, eine C₂-C₄ Dihydroxyalkylgruppe, eine -CH=CHR6-Gruppe, eine -(CH₂)ₚ-CO₂R7-Gruppe oder eine -(CH₂)ₚ-R8-Gruppe mit p= 1,2,3 oder 4, eine -C(R9)=NR10-Gruppe oder eine C(R12)H-NR13R14-Gruppe bedeuten, oder zwei nebeneinanderliegende Reste R1 bis R5 eine -O-CH2-O-Brücke bilden;
**R6** gleich Wasserstoff, einer Nitrogruppe, einer CO₂R7-Gruppe oder einer -C(O)CH₃-Gruppe ist;
**R7, R9 und R12** gleich oder verschieden sein können und unabhängig voneinander gleich Wasserstoff oder einer C₁-C₄-Alkylgruppe sind;
**R8** gleich einer Aminogruppe oder einer Nitrilgruppe ist;
**R10, R13 und R14** gleich oder verschieden sein können und unabhängig voneinander gleich Wasserstoff, einer Hydroxygruppe, einer C₁-C₄-Alkylgruppe, einer C₁-C₄-Hydroxyalkylgruppe, einer C₂-C₄-Dihydroxyalkylgruppe oder einem Rest der Formel 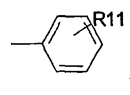 sind;
**R11** gleich Wasserstoff, einer Aminogruppe oder einer Hydroxygruppe ist.

Als Verbindungen der Formel (I) können beispielweise genannt werden: Biphenyl-2,4-diamin; 4-Benzo[1,3]dioxol-5-yl-benzol-1,3-diamin; 2'-(2-Hydroxyethyl)-biphenyl-2,4-diamin; 2'-Acetyl-biphenyl-2,4-diamin; 2'-Amino-biphenyl-2,4-diamin; 2'-Brom-biphenyl-2,4-diamin; 2'-Chlor-biphenyl-2,4-diamin; 2'-Cyan-biphenyl-2,4-diamin; 2'-Ethyl-biphenyl-2,4-diamin; 2'-Fluor-biphenyl-2,4-diamin; 2'-Hydroxy-biphenyl-2,4-diamin; 2'-Methoxy-biphenyl-2,4-diamin; 2'-Methyl-biphenyl-2,4-diamin; 2'-Methylsulfanyl-biphenyl-2,4-diamin; 2'-Nitro-biphenyl-2,4-diamin; 2'-Trifluormethyl-biphenyl-2,4-diamin; 3'-(2-Hydroxyethyl)-biphenyl-2,4-diamin; 3'-Acetyl-biphenyl-2,4-diamin; 3'-Amino-biphenyl-2,4-diamin; 3'-Brom-biphenyl-2,4-diamin; 3'-Chlor-biphenyl-2,4-diamin; 3'-Cyan-biphenyl-2,4-diamin; 3'-Ethyl-biphenyl-2,4-diamin; 3'-Fluor-biphenyl-2,4-diamin; 3'-Hydroxy-biphenyl-2,4-diamin; 3'-Methoxy-biphenyl-2,4-diamin; 3'-Methyl-biphenyl-2,4-diamin; 3'-Methylsulfanyl-biphenyl-2,4-diamin; 3'-Nitro-biphenyl-2,4-diamin; 3'-Trifluormethyl-biphenyl-2,4-diamin; 4'-(2-Hydroxyethyl)-biphenyl-2,4-diamin; 4'-Acetyl-biphenyl-2,4-diamin; 4'-Amino-biphenyl-2,4-diamin; 4'-Brom-biphenyl-2,4-diamin; 4'-Chlor-biphenyl-2,4-diamin; 4'-Cyan-biphenyl-2,4-diamin; 4'-Ethyl-biphenyl-2,4-diamin; 4'-Fluor-biphenyl-2,4-diamin; 4'-Hydroxy-biphenyl-2,4-diamin; 4'-Methoxy-biphenyl-2,4-diamin; 4'-Methyl-biphenyl-2,4-diamin; 4'-Methylsulfanyl-biphenyl-2,4-diamin; 4'-Nitro-biphenyl-2,4-diamin; 4'-Trifluormethyl-biphenyl-2,4-diamin; 2'-Amino-3'-amino-biphenyl-2,4-diamin; 2'-Amino-3'-hydroxy-biphenyl-2,4-diamin; 2'-Amino-3'-methoxy-biphenyl-2,4-diamin; 2'-Amino-3'-methyl-biphenyl-2,4-diamin; 2'-Amino-4'-amino-biphenyl-2,4-diamin; 2'-Amino-4'-hydroxy-biphenyl-2,4-diamin; 2'-Amino-4'-methoxy-biphenyl-2,4-diamin; 2'-Amino-4'-methyl-biphenyl-2,4-diamin; 2'-Amino-5'-amino-biphenyl-2,4-diamin; 2'-Amino-5'-hydroxybiphenyl-2,4-diamin; 2'-Amino-5'-methoxy-biphenyl-2,4-diamin; 2'-Amino-5'-methyl-biphenyl-2,4-diamin; 2'-Amino-6'-amino-biphenyl-2,4-diamin; 2'-Amino-6'-hydroxy-biphenyl-2,4-diamin; 2'-Amino-6'-methoxy-biphenyl-2,4-diamin; 2'-Amino-6'-methyl-biphenyl-2,4-diamin; 2'-Hydroxy-3'-aminobiphenyl-2,4-diamin; 2'-Hydroxy-3'-hydroxy-biphenyl-2,4-diamin; 2'-Hydroxy-3'-methoxy-biphenyl-2,4-diamin; 2'-Hydroxy-3'-methyl-biphenyl-2,4-diamin; 2'-Hydroxy-4'-amino-biphenyl-2,4-diamin; 2'-Hydroxy-4'-hydroxy-biphenyl-2,4-diamin; 2'-Hydroxy-4'-methoxy-biphenyl-2,4-diamin; 2'-Hydroxy-4'-methyl-biphenyl-2,4-diamin; 2'-Hydroxy-5'-amino-biphenyl-2,4-diamin; 2'-Hydroxy-5'-hydroxy-biphenyl-2,4-diamin; 2'-Hydroxy-5'-methoxy-biphenyl-2,4-diamin; 2'-Hydroxy-5'-methyl-biphenyl-2,4-diamin; 2'-Hydroxy-6'-amino-biphenyl-2,4-diamin; 2'-Hydroxy-6'-hydroxy-biphenyl-2,4-diamin; 2'-Hydroxy-6'-methoxy-biphenyl-2,4-diamin; 2'-Hydroxy-6'-methyl-biphenyl-2,4-diamin; 2'-Methoxy-3'-amino-biphenyl-2,4-diamin; 2'-Methoxy-3'-hydroxybiphenyl-2,4-diamin; 2'-Methoxy-3'-methoxy-biphenyl-2,4-diamin; 2'-Methoxy-3'-methyl-biphenyl-2,4-diamin; 2'-Methoxy-4'-amino-biphenyl-2,4-diamin; 2'-Methoxy-4'-hydroxy-biphenyl-2,4-diamin; 2'-Methoxy-4'-methoxy-biphenyl-2,4-diamin; 2'-Methoxy-4'-methyl-biphenyl-2,4-diamin; 2'-Methoxy-5'-amino-biphenyl-2,4-diamin; 2'-Methoxy-5'-hydroxybiphenyl-2,4-diamin; 2'-Methoxy-5'-methoxy-biphenyl-2,4-diamin; 2'-Methoxy-5'-methyl-biphenyl-2,4-diamin; 2'-Methoxy-6'-amino-biphenyl-2,4-diamin; 2'-Methoxy-6'-hydroxy-biphenyl-2,4-diamin; 2'-Methoxy-6'-methoxy-biphenyl-2,4-diamin; 2'-Methoxy-6'-methyl-biphenyl-2,4-diamin; 2'-Methyl-3'-amino-biphenyl-2,4-diamin; 2'-Methyl-3'-hydroxy-biphenyl-2,4-diamin; 2'-Methyl-3'-methoxy-biphenyl-2,4-diamin; 2'-Methyl-3'-methyl-biphenyl-2,4-diamin; 2'-Methyl-4'-amino-biphenyl-2,4-diamin; 2'-Methyl-4'-hydroxy-biphenyl-2,4-diamin; 2'-Methyl-4'-methoxy-biphenyl-2,4-diamin; 2'-Methyl-4'-methyl-biphenyl-2,4-diamin; 2'-Methyl-5'-aminobiphenyl-2,4-diamin; 2'-Methyl-5'-hydroxy-biphenyl-2,4-diamin; 2'-Methyl-5'-methoxy-biphenyl-2,4-diamin; 2'-Methyl-5'-methyl-biphenyl-2,4-diamin; 2'-Methyl-6'-amino-biphenyl-2,4-diamin; 2'-Methyl-6'-hydroxy-biphenyl-2,4-diamin; 2'-Methyl-6'-methoxy-biphenyl-2,4-diamin; 2'-Methyl-6'-methyl-biphenyl-2,4-diamin; 3'-Amino-4'-amino-biphenyl-2,4-diamin; 3'-Amino-4'-hydroxy-biphenyl-2,4-diamin; 3'-Amino-4'-methoxy-biphenyl-2,4-diamin; 3'-Amino-4'-methyl-biphenyl-2,4-diamin; 3'-Amino-5'-aminobiphenyl-2,4-diamin; 3'-Amino-5'-hydroxy-biphenyl-2,4-diamin; 3'-Amino-5'-methoxy-biphenyl-2,4-diamin; 3'-Amino-5'-methyl-biphenyl-2,4-diamin; 3'-Hydroxy-4'-amino-biphenyl-2,4-diamin; 3'-Hydroxy-4'-hydroxy-biphenyl-2,4-diamin; 3'-Hydroxy-4'-methoxy-biphenyl-2,4-diamin; 3'-Hydroxy-4'-methyl-biphenyl-2,4-diamin; 3'-Hydroxy-5'-amino-biphenyl-2,4-diamin; 3'-Hydroxy-5'-hydroxy-biphenyl-2,4-diamin; 3'-Hydroxy-5'-methoxy-biphenyl-2,4-diamin; 3'-Hydroxy-5'-methyl-biphenyl-2,4-diamin; 3'-Methoxy-4'-amino-biphenyl-2,4-diamin; 3'-Methoxy-4'-hydroxy-biphenyl-2,4-diamin; 3'-Methoxy-4'-methoxy-biphenyl-2,4-diamin; 3'-Methoxy-4'-methyl-biphenyl-2,4-diamin; 3'-Methoxy-5'-amino-biphenyl-2,4-diamin; 3'-Methoxy-5'-hydroxy-biphenyl-2,4-diamin; 3'-Methoxy-5'-methoxy-biphenyl-2,4-diamin; 3'-Methoxy-5'-methyl-biphenyl-2,4-diamin; 3'-Methyl-4'-amino-biphenyl-2,4-diamin; 3'-Methyl-4'-hydroxy-biphenyl-2,4-diamin; 3'-Methyl-4'-methoxy-biphenyl-2,4-diamin; 3'-Methyl-4'-methyl-biphenyl-2,4-diamin; 3'-Methyl-5'-amino-biphenyl-2,4-diamin; 3'-Methyl-5'-hydroxybiphenyl-2,4-diamin; 3'-Methyl-5'-methoxy-biphenyl-2,4-diamin und 3'-Methyl-5'-methyl-biphenyl-2,4-diamin, sowie deren physiologisch verträglichen Salze.

Bevorzugt sind Verbindungen der Formel (I) oder deren physiologisch verträgliche Salze, in denen (i) mindestens 3 der Reste **R1** bis **R5** Wasserstoff bedeuten oder (ii) 3 der Reste **R1** bis **R5** gleich Wasserstoff sind und die beiden verbleibenden Reste unabhängig voneinander Wasserstoff, eine Methoxygruppe, eine Hydroxygruppe, eine C₁-C₄-Alkylgruppe, eine C₁-C₄-Hydroxyalkylgruppe oder eine Aminogruppe darstellen, oder -wenn die beiden verbleibenden Reste nebeneinanderliegen- gemeinsam eine -O-CH2-O-Brücke bilden.

Besonders bevorzugt sind die folgenden [1,1'-Biphenyl]-2,4-diamin-Derivate der Formel (I): Biphenyl-2,4-diamin; 4-Benzo[1,3]dioxol-5-yl-benzol-1,3-diamin, 4'-Hydroxy-biphenyl-2,4-diamin; 4'-Amino-biphenyl-2,4-diamin; 3'-Hydroxy-biphenyl-2,4-diamin; 4'-Methoxy-biphenyl-2,4-diamin sowie deren physiologisch verträglichen Salze.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die [1,1'-Biphenyl]-2,4-diamin-Derivate der Formel (1) sind in dem erfindungsgemäßen Färbemittel in einer Gesamtmenge von etwa 0,005 bis 20 Gewichtsprozent enthalten, wobei eine Menge von etwa 0,01 bis 5 Gewichtsprozent und insbesondere 0,1 bis 2,5 Gewichtsprozent bevorzugt ist.

Als Entwicklersubstanzen können alle für derartige Färbemittel bekannten und geeigneten Entwicklersubstanzen, beispielsweise 1,4-Diaminobenzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 1-(2,5-Diamino-phenyl)-ethanol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyethyl)-benzol, 1,4-Diamino-2-(1-methyl-ethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methylphenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1 H-pyrazol, 4,5-Diamino-1-(1-methyl-ethyl)-1 H-pyrazol, 4,5-Diamino-1-[(4-methylphenyl)methyl]-1H-pyrazol, 1-[(4-Chlorphenyl)methyl]-4.,5-diamino-1 H-pyrazol, 4,5-Diamino-1-methyl-1H-pyrazol, 4,5-Diamino-3-methyl-1-methyl-1 H-pyrazol, 4,5-Diamino-3-methyl-1 -methyl-1 H-pyrazol, 2-Amino-phenol, 2-Amino-6-methyl-phenol und 2-Amino-5-methyl-phenol,eingesetzt werden.

Weiterhin kann das erfindungsgemäße Färbemittel gegebenenfalls zusätzlich weitere bekannte Kupplersubstanzen, beispielsweise 2,6-Diamino-pyridin, 2-Amino-4-[((2-hydroxyethyl))amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methylphenol, 5-Amino-4-methoxy-2-methyl-phenol, 5-Amino-4-ethoxy-2-methylphenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methylphenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol,7-Hydroxy-indol und 2,3-Indolindion, enthalten.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt. Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Überschuß oder Unterschuß (beispielsweise in einem Verhältnis (Kuppler: Entwickler) von 1:2 bis 1:0,5) vorhanden sind.

Weiterhin kann das erfindungsgemäße Färbemittel zusätzlich andere Farbkomponenten, beispielsweise 6-Amino-2-methylphenol und 2-Amino-5-methylphenol, sowie ferner übliche anionische, kationische, zwitterionische oder nicht-ionische direktziehende Farbstoffe, beispielsweise Triphenylmethanfarbstoffe wie 4-[(4'-aminophenyl)-(4'imino-2",5"-cyclohexadien-1 "-yliden)-methyl]-2-methylaminobenzol-monohydrochlorid (C.I. 42 510) und 4-[(4'amino-3'-methyl-phenyl)-(4"-imino-3"-methyl-2",5"cyclohexadien-1 "-yliden)-methyl]-2-methyl-aminobenzol monohydrochlorid (C.I. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5-hydroxynaphthalin-1-sulfonsäure-Natriumsalz (C.l. 14 805) und Dispersionsfarbstoffe wie beispielsweise 1,4-Diaminoanthrachinon und 1,4,5,8-Tetraamino-antrachinon, enthalten. Die vorgenannten Farbkomponenten können in dem erfindungsgemäßen Färbemittel in einer Menge von etwa 0,1 bis 4 Gewichtsprozent enthalten sein.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in dem erfindungsgemäßen Färbemittel, falls dieses zur Färbung von Haaren verwendet werden soll, noch weitere für kosmetische Mittel übliche Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 50 bis 200 Gramm, dieses Gemisches auf das Haar auf. Das nach dem Vermischen mit dem Oxidationsmittel erhaltene gebrauchsfertige Oxidationshaarfärbemittel hat vorzugsweise einen pH-Wert von 6,5 bis 11,5.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3- bis 12-prozentigen, vorzugsweise 6-prozentigen, wässrigen Lösung, aber auch Luftsauerstoff in Betracht. Wird eine 6-prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschliessend wird das Haar getrocknet.

Das erfindungsgemäße Färbemittel mit einem Gehalt an [1,1'-Biphenyl]-2,4-diamin-Derivaten der Formel (I) als Kupplersubstanz ermöglicht Färbungen mit ausgezeichneter Farbechtheit, insbesondere was die Lichtechtheit, Waschechtheit und Reibeechtheit anbetrifft. Hinsichtlich der färberischen Eigenschaften bietet das erfindungsgemäße Färbemittel je nach Art und Zusammensetzung der Farbkomponenten eine breite Palette verschiedener Farbnuancen, welche sich je nach verwendeter Entwicklersubstanz-Kupplersubstanz-Kombination von blonden über braune, purpurne, violette bis hin zu blauen und schwarzen Farbtönen erstreckt, wobei insbesondere die nunmher erzielbaren intensiven und stabilen blauen Farbnuancen hervorzuheben sind. Die sehr guten färberischen Eigenschaften des Färbemittels gemäß der vorliegenden Anmeldung zeigen sich weiterhin darin, daß dieses Mittel insbesondere auch eine Anfärbung von ergrauten, chemisch nicht vorgeschädigten Haaren problemlos und mit guter Deckkraft ermöglicht.

Die Herstellung der erfindungsgemäßen [1,1'-Biphenyl]-2,4-diamin-Derivate der Formel (I) kann unter Verwendung von bekannten Syntheseverfahren, beispielsweise in Analogie zu den in den Ausführungsbeispielen beschriebenen Verfahren, erfolgen.

Die [1,1'-Biphenyl]-2,4-diamin-Derivate der Formel (I) sind gut in Wasser löslich und ermöglichen Färbungen -insbesondere im blauen und purpurnen Farbbereich- mit hoher Farbintensität und ausgezeichneter Farbechtheit, insbesondere was die Lichtechtheit, Waschechtheit und Reibeechtheit anbetrifft. Sie weisen weiterhin eine ausgezeichnete Lagerstabilität, insbesondere als Bestandteil der hier beschriebenen Oxidationsfärbemitteln, auf.

Ein weiterer Gegenstand der vorliegenden Erfindung sind daher [1,1'-Biphenyl]-2,4-diamin-Derivate der allgemeinen Formel (I), 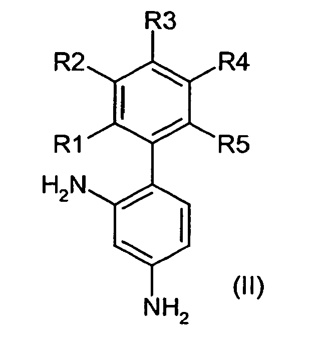 welche ausgewählt sind aus der Gruppe bestehend aus 4-Benzo[1,3]dioxol-5-yl-benzol-1,3-diamin; 2'-(2-Hydroxyethyl)-biphenyl-2,4-diamin; 2'-Acetyl-biphenyl-2,4-diamin; 2'-Amino-biphenyl-2,4-diamin; 2'-Brom-biphenyl-2,4-diamin; 2'-Chlor-biphenyl-2,4-diamin; 2'-Cyano-biphenyl-2,4-diamin; 2'-Ethyl-biphenyl-2,4-diamin; 2'-Fluor-biphenyl-2,4-diamin; 2'-Hydroxy-biphenyl-2,4-diamin; 2'-Methoxy-biphenyl-2,4-diamin; 2'-Methyl-biphenyl-2,4-diamin; 2'-Methylsulfanyl-biphenyl-2,4-diamin; 2'-Nitro-biphenyl-2,4-diamin; 2'-Trifluormethyl-biphenyl-2,4-diamin; 3'-(2-Hydroxyethyl)-biphenyl-2,4-diamin; 3'-Acetyl-biphenyl-2,4-diamin; 3'-Amino-biphenyl-2,4-diamin; 3'-Brom-biphenyl-2,4-diamin; 3'-Chlor-biphenyl-2,4-diamin; 3'-Cyano-biphenyl-2,4-diamin; 3'-Ethyl-biphenyl-2,4-diamin; 3'-Fluor-biphenyl-2,4-diamin; 3'-Hydroxy-biphenyl-2,4-diamin; 3'-Methoxy-biphenyl-2,4-diamin; 3'-Methyl-biphenyl-2,4-diamin; 3'-Methylsulfanyl-biphenyl-2,4-diamin; 3'-Nitro-biphenyl-2,4-diamin; 3'-Trifluormethyl-biphenyl-2,4-diamin; 4'-(2-Hydroxyethyl)-biphenyl-2,4-diamin; 4'-Acetyl-biphenyl-2,4-diamin; 4'-Amino-biphenyl-2,4-diamin; 4'-Brom-biphenyl-2,4-diamin; 4'-Chlor-biphenyl-2,4-diamin; 4'-Cyano-biphenyl-2,4-diamin; 4'-Ethyl-biphenyl-2,4-diamin; 4'-Fluor-biphenyl-2,4-diamin; 4'-Hydroxy-biphenyl-2,4-diamin; 4'-Methoxy-biphenyl-2,4-diamin; 4'-Methyl-biphenyl-2,4-diamin; 4'-Methylsulfanyl-biphenyl-2,4-diamin; 4'-Nitro-biphenyl-2,4-diamin; 4'-Trifluormethyl-biphenyl-2,4-diamin; 2'-Amino-3'-amino-biphenyl-2,4-diamin; 2'-Amino-3'-hydroxy-biphenyl-2,4-diamin; 2'-Amino-3'-methoxy-biphenyl-2,4-diamin; 2'-Amino-3'-methyl-biphenyl-2,4-diamin; 2'-Amino-4'-amino-biphenyl-2,4-diamin; 2'-Amino-4'-hydroxy-biphenyl-2,4-diamin; 2'-Amino-4'-methoxy-biphenyl-2,4-diamin; 2'-Amino-4'-methyl-biphenyl-2,4-diamin; 2'-Amino-5'-amino-biphenyl-2,4-diamin; 2'-Amino-5'-hydroxy-biphenyl-2,4-diamin; 2'-Amino-5'-methoxy-biphenyl-2,4-diamin; 2'-Amino-5'-methyl-biphenyl-2,4-diamin; 2'-Amino-6'-amino-biphenyl-2,4-diamin; 2'-Amino-6'-hydroxy-biphenyl-2,4-diamin; 2'-Amino-6'-methoxy-biphenyl-2,4-diamin; 2'-Amino-6'-methyl-biphenyl-2,4-diamin; 2'-Hydroxy-3'-amino-biphenyl-2,4-diamin; 2'-Hydroxy-3'-hydroxy-biphenyl-2,4-diamin; 2'-Hydroxy-3'-methoxy-biphenyl-2,4-diamin; 2'-Hydroxy-3'-methyl-biphenyl-2,4-diamin; 2'-Hydroxy-4'-amino-biphenyl-2,4-diamin; 2'-Hydroxy-4'-hydroxy-biphenyl-2,4-diamin; 2'-Hydroxy-4'-methoxy-biphenyl-2,4-diamin; 2'-Hydroxy-4'-methyl-biphenyl-2,4-diamin; 2'-Hydroxy-5'-amino-biphenyl-2,4-diamin; 2'-Hydroxy-5'-hydroxy-biphenyl-2,4-diamin; 2'-Hydroxy-5'-methoxy-biphenyl-2,4-diamin; 2'-Hydroxy-5'-methyl-biphenyl-2,4-diamin; 2'-Hydroxy-6'-amino-biphenyl-2,4-diamin; 2'-Hydroxy-6'-hydroxy-biphenyl-2,4-diamin; 2'-Hydroxy-6'-methoxy-biphenyl-2,4-diamin; 2'-Hydroxy-6'-methyl-biphenyl-2,4-diamin; 2'-Methoxy-3'-amino-biphenyl-2,4-diamin; 2'-Methoxy-3'-hydroxy-biphenyl-2,4-diamin; 2'-Methoxy-3'-methoxy-biphenyl-2,4-diamin; 2'-Methoxy-3'-methyl-biphenyl-2,4-diamin; 2'-Methoxy-4'-amino-biphenyl-2,4-diamin; 2'-Methoxy-4'-hydroxy-biphenyl-2,4-diamin; 2'-Methoxy-4'-methoxy-biphenyl-2,4-diamin; 2'-Methoxy-4'-methyl-biphenyl-2,4-diamin; 2'-Methoxy-5'-amino-biphenyl-2,4-diamin; 2'-Methoxy-5'-hydroxy-biphenyl-2,4-diamin; 2'-Methoxy-5'-methoxy-biphenyl-2,4-diamin; 2'-Methoxy-5'-methyl-biphenyl-2,4-diamin; 2'-Methoxy-6'-amino-biphenyl-2,4-diamin; 2'-Methoxy-6'-hydroxy-biphenyl-2,4-diamin; 2'-Methoxy-6'-methoxy-biphenyl-2,4-diamin; 2'-Methoxy-6'-methyl-biphenyl-2,4-diamin; 2'-Methyl-3'-amino-biphenyl-2,4-diamin; 2'-Methyl-3'-hydroxy-biphenyl-2,4-diamin; 2'-Methyl-3'-methoxy-biphenyl-2,4-diamin; 2'-Methyl-3'-methyl-biphenyl-2,4-diamin; 2'-Methyl-4'-amino-biphenyl-2,4-diamin; 2'-Methyl-4'-hydroxybiphenyl-2,4-diamin; 2'-Methyl-4'-methoxy-biphenyl-2,4-diamin; 2'-Methyl-4'-methyl-biphenyl-2,4-diamin; 2'-Methyl-5'-amino-biphenyl-2,4-diamin; 2'-Methyl-5'-hydroxy-biphenyl-2,4-diamin; 2'-Methyl-5'-methoxy-biphenyl-2,4-diamin; 2'-Methyl-5'-methyl-biphenyl-2,4-diamin; 2'-Methyl-6'-aminobiphenyl-2,4-diamin; 2'-Methyl-6'-hydroxy-biphenyl-2,4-diamin; 2'-Methyl-6'-methoxy-biphenyl-2,4-diamin; 2'-Methyl-6'-methyl-biphenyl-2,4-diamin; 3'-Amino-4'-hydroxy-biphenyl-2,4-diamin; 3'-Amino-4'-methoxy-biphenyl-2,4-diamin; 3'-Amino-4'-methyl-biphenyl-2,4-diamin; 3'-Amino-5'-aminobiphenyl-2,4-diamin; 3'-Amino-5'-hydroxy-biphenyl-2,4-diamin; 3'-Amino-5'-methoxy-biphenyl-2,4-diamin; 3'-Amino-5'-methyl-biphenyl-2,4-diamin; 3'-Hydroxy-4'-amino-biphenyl-2,4-diamin; 3'-Hydroxy-4'-hydroxy-biphenyl-2,4-diamin; 3'-Hydroxy-4'-methoxy-biphenyl-2,4-diamin; 3'-Hydroxy-4'-methyl-biphenyl-2,4-diamin; 3'-Hydroxy-5'-amino-biphenyl-2,4-diamin; 3'-Hydroxy-5'-hydroxy-biphenyl-2,4-diamin; 3'-Hydroxy-5'-methoxy-biphenyl-2,4-diamin; 3'-Hydroxy-5'-methyl-biphenyl-2,4-diamin; 3'-Methoxy-4'-amino-biphenyl-2,4-diamin; 3'-Methoxy-4'-hydroxy-biphenyl-2,4-diamin; 3'-Methoxy-4'-methoxy-biphenyl-2,4-diamin; 3'-Methoxy-4'-methyl-biphenyl-2,4-diamin; 3'-Methoxy-5'-amino-biphenyl-2,4-diamin; 3'-Methoxy-5'-hydroxy-biphenyl-2,4-diamin; 3'-Methoxy-5'-methoxy-biphenyl-2,4-diamin; 3'-Methoxy-5'-methyl-biphenyl-2,4-diamin; 3'-Methyl-4'-amino-biphenyl-2,4-diamin; 3'-Methyl-4'-hydroxy-biphenyl-2,4-diamin; 3'-Methyl-4'-methoxy-biphenyl-2,4-diamin; 3'-Methyl-4'-methyl-biphenyl-2,4-diamin; 3'-Methyl-5'-amino-biphenyl-2,4-diamin; 3'-Methyl-5'-hydroxybiphenyl-2,4-diamin; 3'-Methyl-5'-methoxy-biphenyl-2,4-diamin und 3'-Methyl-5'-methyl-biphenyl-2,4-diamin, sowie deren physiologisch verträglichen Salzen.

Die nachfolgenden Beispiele sollen den Gegenstand der Erfindung näher erläutern, ohne ihn darauf zu beschränken.

### Beispiele

### Beispiel 1:

### A. Synthese von (4-Brom-3-tert-butoxycarbonylamino-phenyl)-carbaminsäure-tert.butylester

Zu einer Suspension von 10 g (32,4 mmol) 3-tert-Butoxycarbonylaminophenyl)-carbaminsäure-tert.butylester in 100 ml 1,2-Dimethoxyethan tropft man bei 0 °C innerhalb von 2 Stunden eine Lösung von 6 g (33,7 mmol) N-Brom-succinimid in 50 ml 1,2-Dimethoxyethan. Die Reaktionsmischung wird für 2 Stunde weitergerührt. Nach Beendigung der Reaktion wird die Reaktionsmischung in 300 ml Wasser gegossen, wobei sich ein Niederschlag bildet. Der Niederschlag wird abfiltriert und mit Wasser gewaschen.
Es werden 11 g (94 % der Theorie) (4-Brom-3-tert-butoxycarbonylaminophenyl)-carbaminsäure-tert.butylester erhalten.
¹H-NMR (300 MHz, DMSO-D6): δ= 9,51 (s, 1 H), 8,43 (s, 1 H), 7,89 (s, 1 H), 7,47 (d; 1 H), 7,18 (d, 1 H), 1,47 (d, 18H)

### B. Synthese von [3-tert-Butoxycarbonylamino-4-(4,4,5,5-tetramethyl-[1,3,2]dioxaborolan-2-yl)-phenyl]-carbaminsäure-tert.butylester

Eine Mischung von 7,8 g (20,2 mmol) (4-Brom-3-tert-butoxycarbonylamino-phenyl)-carbaminsäure-tert.butylester aus Stufe A, 12,8 g (50,6 mmol) 4,4,4',4',5,5,5,5'-Octamethyl-[2,2']bi[[1,3,2]dioxaborinanyl], 2 g (2,9 mmol) Dichloro-(1,1'-bis(diphenyl-phosphino)ferrocene)palladium (PdCl₂(dppf)) und 6,2 g (63,2 mmol) Kaliumacetat werden unter Argon mit 210 ml entgastem Dioxan versetzt. Das Gemisch wird 26 Stunden lang bei 80 °C gerührt und anschliessend mit einem Gemisch aus 4,2 g (16,9 mmol) Diboronpinacolester und 700 mg (0,95 mmol) PdCl₂(dppf) versetzt. Das Reaktionsgemisch wird weitere 14 Stunden lang bei 80 °C gerührt, sodann auf Wasser gegossen und mit Essigsäureethylester extrahiert. Die organische Phase wird anschliessend mit einer gesättigten wässerigen Kochsalz-Lösung gewaschen, über Natriumsulfat getrocknet und nach Filtration eingeengt. Das erhaltene Rohprodukt wird an desaktiviertem Kieselgel mit Hexan/Essigsäure-ethylester (1:1) gereinigt.
Es werden 6,20 g (71% der Theorie) 3-tert-Butoxycarbonylamino-4-(4,4,5,5-tetramethyl-[1,3,2]dioxaborolan-2-yl)-phenyl]-carbaminsäure-tert.butylester erhalten.

### C. Synthese von [1,1'-Biphenyl]-2,4-diaminen

0,09 g (0,0002 mol) 3-tert-Butoxycarbonylamino-4-(4,4,5,5-tetramethyl-[1,3,2]dioxaborolan-2-yl)-phenyl]-carbaminsäure-tert.butylester aus Stufe **B** und 0,0004 mol des entsprechenden Bromderivates werden unter Argon in 10 ml 1,2-Dimethoxyethan gelöst. Anschliessend werden 0,01 g Tetrakis-(triphenylphosphin)-palladium (0,000005 mol) und 0,26 ml 2N Kaliumcarbonatlösung zugegeben und die Reaktionsmischung auf 80 °C erwärmt. Nach Beendigung der Reaktion wird die Reaktionsmischung in 10 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol auf 50 °C erwärmt. Anschliessend werden zur Herstellung des Hydrochlorides 1,5 ml einer 2,9 molaren ethanolische Salzsäurelösung zugetropft. Der Niederschlag wird abfiltriert, zweimal mit 1 ml Ethanol gewaschen und sodann getrocknet.
**a.** Biphenyl-2,4,4'-triamin Hydrochlorid
   Verwendetes Bromderivat: 4-Brom-anilin
   Massenspektrum: MH+ 200(100)
**b.** 2',4'-Diamino-biphenyl-4-ol Hydrochlorid
   Verwendetes Bromderivat: 4-Brom-phenol
   Massenspektrum: MH+ 201(15)
**c.** 2',4'-Diamino-biphenyl-3-ol Hydrochlorid
   Verwendetes Bromderivat: 3-Brom-phenol
   Massenspektrum: MH+ 201(40)
**d.** Biphenyl-2,4-diamin Hydrochlorid
   Verwendetes Bromderivat: 4-Brom-benzol
   Massenspektrum: MH+ 185(100)
**e.** 3'-Methoxy-biphenyl-2,4-diamin Hydrochlorid
   Verwendetes Bromderivat: 3-Brom-anisol
   Massenspektrum: MH+ 215(60)
**f.** 4-Benzo[1,3]dioxol-5-yl-benzol-1,3-diamin Hydrochlorid
   Verwendetes Bromderivat: 5-Bromo-benzo[1,3]dioxol
   Massenspektrum: MH+ 229(90)
**g.** 4'-Methyl-biphenyl-2,4-diamin Hydrochlorid
   Verwendetes Bromderivat: 4-Brom-toluol
   Massenspektrum: MH+ 199(100)
**h.** 4'-Fluor-biphenyl-2,4-diamin Hydrochlorid
   Verwendetes Bromderivat: 4-Fluor-1-brombenzol
   Massenspektrum: MH+ 23(100)
**i**. 2',4'-Diamino-biphenyl-4-carbonitril Hydrochlorid
   Verwendetes Bromderivat: 4-Brom-benzonitril
   Massenspektrum: MH+ 210(15)
**j.** 1-(2',4'-Diamino-biphenyl-3-yl)-ethanon Hydrochlorid
   Verwendetes Bromderivat: 3-Brom-acetophenon
   Massenspektrum: MH+ 227(60)
**k.** 4'-Nitro-biphenyl-2,4-diamin Hydrochlorid
   Verwendetes Bromderivat: 4-Brom-nitrobenzol
   Massenspektrum: MH+ 230(100)
**l.** 2'-Nitro-biphenyl-2,4-diamin Hydrochlorid
   Verwendetes Bromderivat: 2-Brom-nitrobenzol
   Massenspektrum: MH+ 230(100)
**m.** 4'-Trifluormethyl-biphenyl-2,4-diamin Hydrochlorid
   Verwendetes Bromderivat: 4-Brom-trifluormethylbenzol
   Massenspektrum: MH+ 253(20)
**n.** 2',4'-Dimethyl-biphenyl-2,4-diamin Hydrochlorid
   Verwendetes Bromderivat: 2-Brom-m-xylol
   Massenspektrum: MH+ 213(60)
**o.** 4'-Chlor-biphenyl-2,4-diamin Hydrochlorid
   Verwendetes Bromderivat: 4-Brom-chlorbenzol
   Massenspektrum: MH+ 219(28)
**p.** 4'-Methylsulfanyl-biphenyl-2,4-diamin Hydrochlorid
   Verwendetes Bromderivat: 1-Brom-4-methylsulfanyl-benzol Massenspektrum: MH+ 231(18)

### Beispiele 2 bis 17: Haarfärbemittel

Es werden Haarfärbelösungen der folgenden Zusammensetzung hergestellt:

| | |
|---|---|
| 1,25 mmol | Kupplersubstanz der Formel (I) gemäß Tabelle 1 |
| 1,25 mmol | Entwicklersubstanz gemäß Tabelle 1 |
| 1,0 g | Kaliumoleat (8-prozentige wässrige Lösung) |
| 1,0 g | Ammoniak (22-prozentige wässrige Lösung) |
| 1,0 g | Ethanol |
| 0,3 g | Ascorbinsäure |
| ad 100,0 g | Wasser |

50 g der vorstehenden Färbelösung werden unmittelbar vor der Anwendung mit 50 g einer 6-prozentigen wässrigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefasst.

**Tabelle 1:**

| **Beispiel Nr.** | **Kupplersubstanz der Formel (I)** | **Kupplersubstanz** | | | |
|---|---|---|---|---|---|
| | | **I.** | **II.** | **III.** | **IV.** |
| | | **1,4-Diaminobenzol** | **2,5-Diaminotoluol-sulfat** | **2,5-Diamino-phenyl-ethanol-sulfat** | **4,5-Diamino-1-(2'-hydroxy-ethyl)-pyrazol-sulfat** |
| **2.** | gemäß Beispiel **1a** | dunkelblau | dunkelblau | dunkelblau | purpur |
| **3.** | gemäß Beispiel **1b** | dunkelblau | dunkelblau | dunkelblau | purpur |
| **4.** | gemäß Beispiel **1c** | dunkelblau | dunkelblau | dunkelblau | purpur |
| **5.** | gemäß Beispiel **1d** | dunkelblau | blau | dunkelblau | purpur |
| **6.** | gemäß Beispiel **1e** | dunkelblau | dunkelblau | dunkelblau | purpur |
| **7.** | gemäß Beispiel **1f** | dunkelblau | dunkelblau | dunkelblau | purpur |
| **8.** | gemäß Beispiel **1g** | dunkelblau | dunkelblau | blau | purpur |
| **9.** | gemäß Beispiel **1h** | dunkelblau | dunkelblau | blau | purpur |
| **10.** | gemäß Beispiel **1i** | dunkelblau | blau | blau | purpur |
| **11.** | gemäß Beispiel **1j** | dunkelblau | blau | blau | purpur |
| **12.** | gemäß Beispiel **1k** | dunkelblau | blau | blau | purpur |
| **13.** | gemäß Beispiel **1l** | dunkelblau | blau | blau | purpur |
| **14**. | gemäß Beispiel **1m** | dunkelblau | blau | blau | purpur |
| **15.** | gemäß Beispiel **1n** | dunkelblau | blau | blau | purpur |
| **16.** | gemäß Beispiel **1o** | dunkelblau | blau | blau | purpur |
| **17.** | gemäß Beispiel **1p** | dunkelblau | blau | blau | purpur |

### Beispiele 18 bis 41: Haarfärbemittel

Es werden Haarfärbelösungen der folgenden Zusammensetzung hergestellt:

| | |
|---|---|
| X g | [1,1'-Biphenyl]-2,4-diamin (Kupplersubstanz **K1** bis **K4** der Formel (I) gemäß Tabelle 4) |
| U g | Entwicklersubstanz **E8** bis **E15** gemäß Tabelle 2 |
| Y g | Kupplersubstanz **K11** bis **K36** gemäß Tabelle 4 |
| Z g | direktziehender Farbstoff **D1** bis **D3** gemäß Tabelle 3 |
| 10,0 g | Kaliumoleat (8-prozentige wässrige Lösung) |
| 10,0 g | Ammoniak (22-prozentige wässrige Lösung) |
| 10,0 g | Ethanol |
| 0,3 g | Ascorbinsäure |
| ad 100,0 g | Wasser |

30 g der vorstehenden Färbelösung werden unmittelbar vor der Anwendung mit 30 g einer 6prozentigen wässrigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die Färbeergebnisse sind in Tabelle 5 zusammengefasst.

**Tabelle 2:**

| **Entwicklersubstanzen** | |
|---|---|
| **E8** | 1,4-Diaminobenzol |
| **E9** | 2-(2,5-Diamino-phenyl)-ethanol-sulfat |
| **E10** | 3-Methyl-4-amino-phenol |
| **E11** | 4-Amino-2-aminomethyl-phenol-dihydrochlorid |
| **E12** | 4-Amino-phenol |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 3:**

| **Direktziehende Farbstoffe** | |
|---|---|
| **D1** | 2,6-Diamino-3-((pyridin-3-yl)azo)pyridin |
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K1** | Biphenyl-2,4,4'-triamin Hydrochlorid |
| **K2** | Biphenyl-2,4-diamin Hydrochlorid |
| **K3** | 2',4'-Diamino-biphenyl-4-ol Hydrochlorid |
| **K4** | 2',4'-Diamino-biphenyl-3-ol Hydrochlorid |
| **K11** | 1,3-Diaminobenzol |
| **K12** | 2-Amino-4-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor-toluol-sulfat |
| **K15** | 3-Amino-2-methylamino-6-methoxy-pyridin |
| **K16** | 3,5-Diamino-2,6-dimethoxy-pyridin-dihydrochlorid |
| **K17** | 2,4-Diamino-5-ethoxy-toluol-sulfat |
| **K18** | N-(3-Dimethylamino)phenylhamstoff |
| **K19** | 1,3-Bis(2,4-Diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzol-HCl |
| **K35** | 3,4-Methylendioxy-phenol |
| **K36** | 2-Amino-5-methyl-phenol |

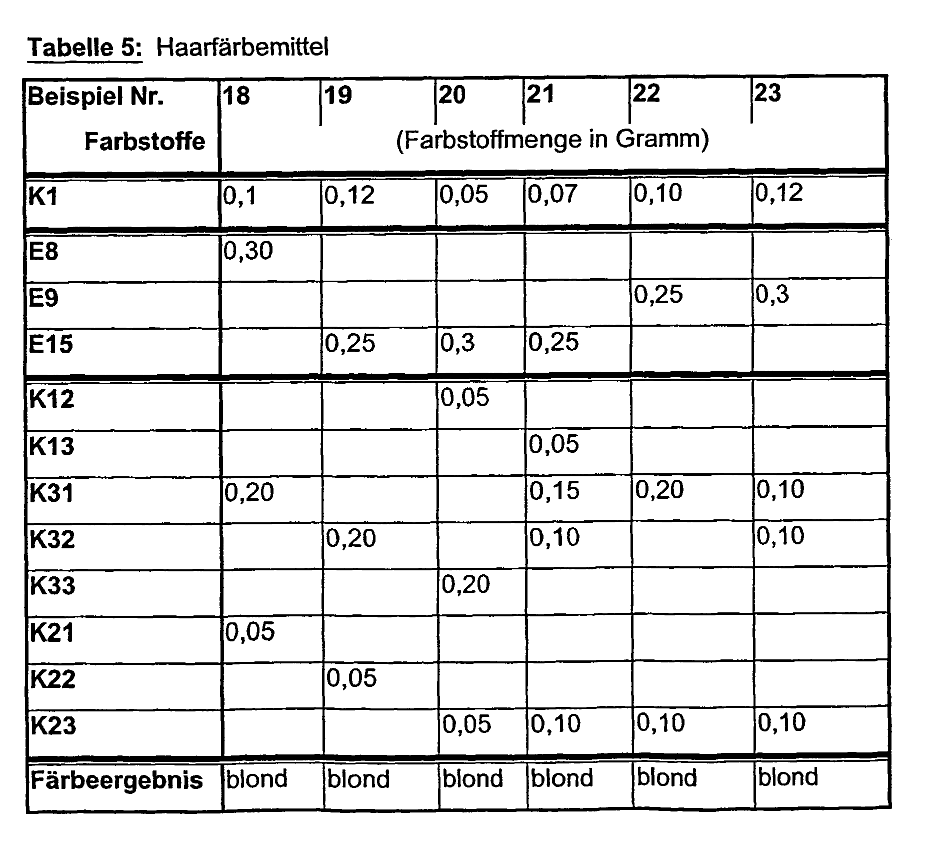

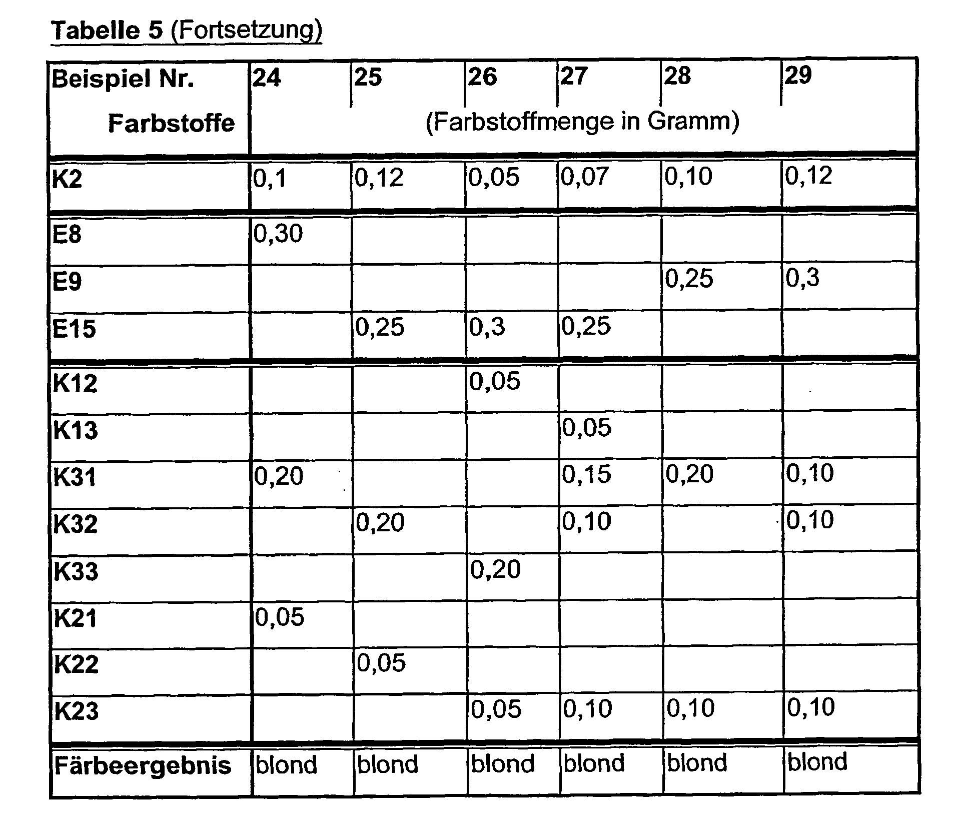

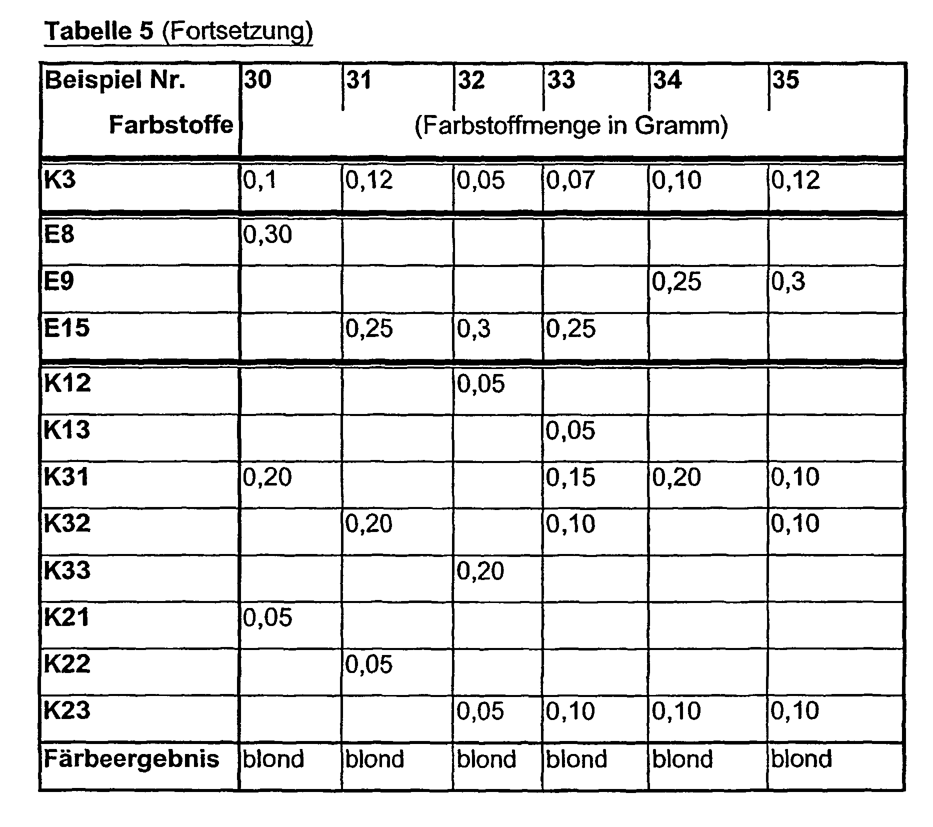

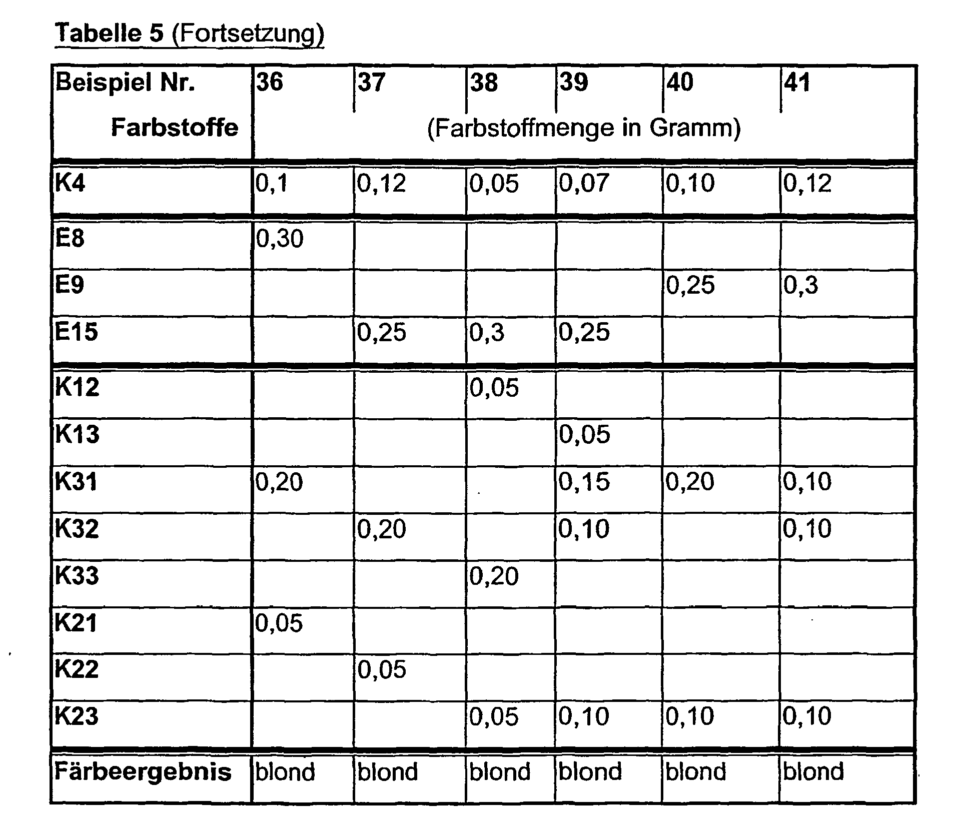

### Beispiele 42 bis 65: Haarfärbemittel

Es werden die folgenden cremeförmigen Farbträgermassen hergestellt:

| | |
|---|---|
| X g | [1,1'-Biphenyl]-2,4-diamin (Kupplersubstanz **K1 bis K4** der Formel (I) gemäß Tabelle 4) |
| U g | Entwicklersubstanz **E8** bis **E15** gemäß Tabelle 2 |
| Y g | Kupplersubstanz **K11** bis **K36** gemäß Tabelle 4 |
| Z g | direktziehender Farbstoff **D2** gemäß Tabelle 3 |
| 15,0 g | Cetylalkohol |
| 0,3 g | Ascorbinsäure |
| 3,5 g | Natriumlaurylalkoholdiglycolethersulfat, 28-prozentige wässrige Lösung |
| 3,0 g | Ammoniak, 22-prozentige wässrige Lösung |
| 0,3 g | Natriumsulfit, wasserfrei |
| ad 100,0 g | Wasser |

30 g der vorstehenden Färbecreme werden unmittelbar vor der Anwendung mit 30 g einer 6-prozentigen Wasserstoffperoxidlösung vermischt. Anschliessend wird das Gemisch auf das Haar aufgetragen. Nach einer Einwirkzeit von 30 Minuten bei 40 °C wird das Haar mit Wasser gespült, mit einem handelsüblichen Shampoo gewaschen und getrocknet. Die Färbeergebnisse sind in Tabelle 6 zusammengefasst.

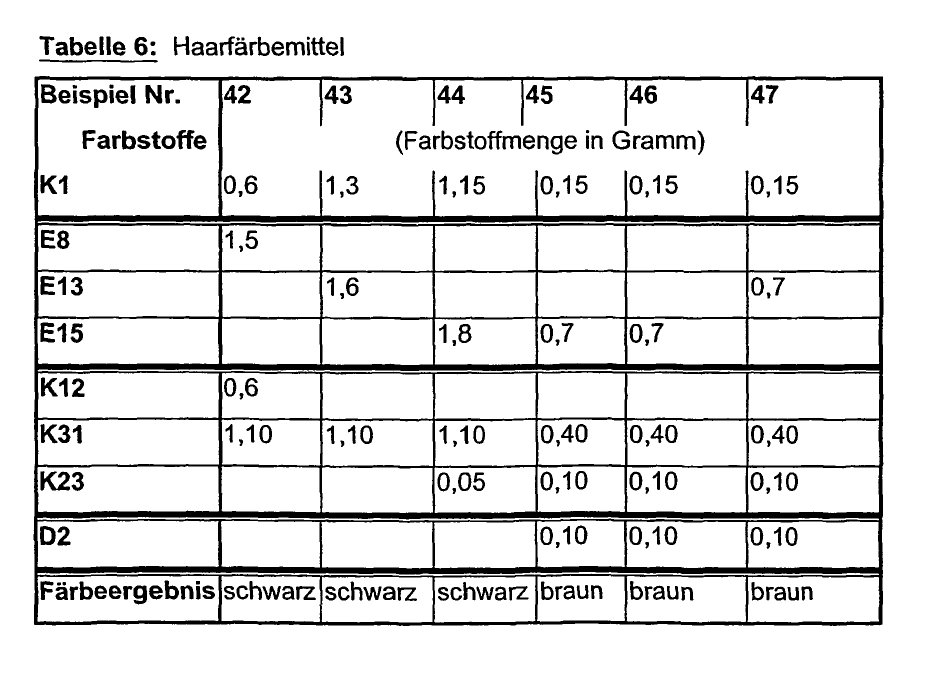

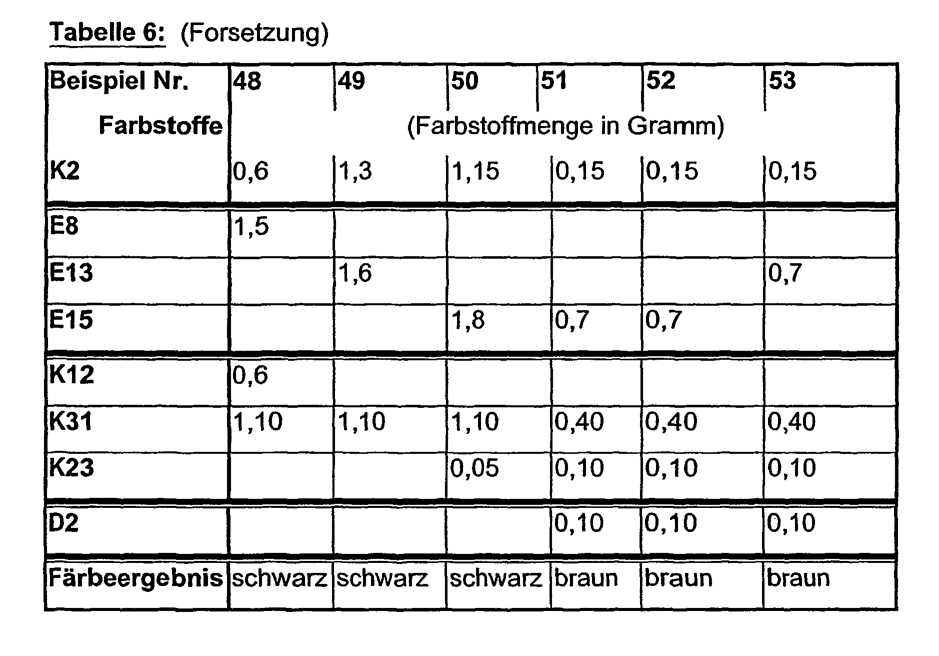

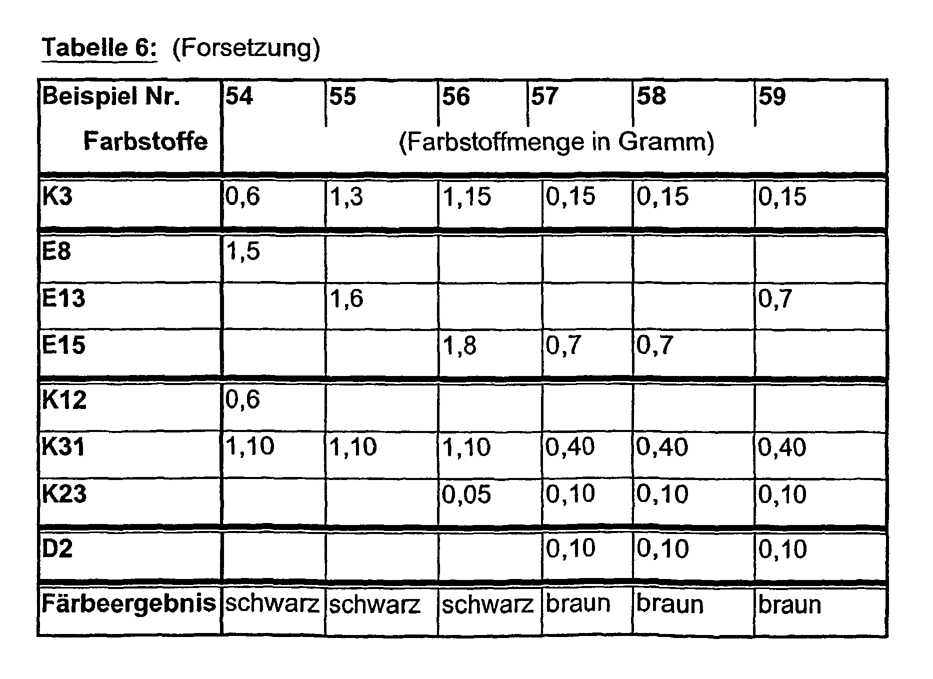

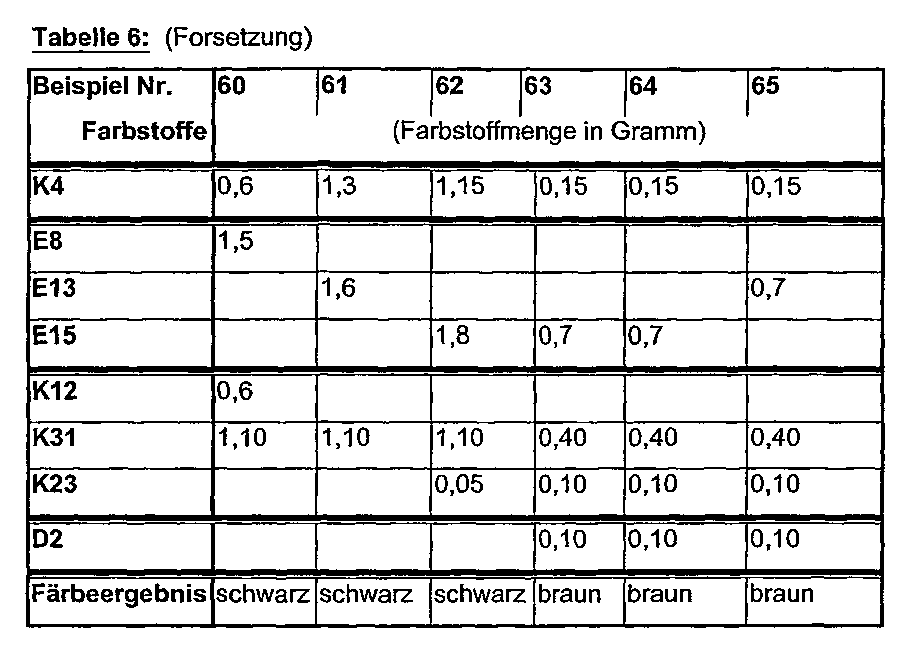

Alle in der vorliegenden Anmeldung enthaltenen Prozentangaben stellen soweit nicht anders angegeben Gewichtsprozente dar.

## Patentansprüche

1. Mittel zur oxidativen Färbung von Keratinfasern auf der Basis einer Entwicklersubstanz-Kupplersubstanz-Kombination, **dadurch gekennzeichnet, dass** es als Kupplersubstanz mindestens ein [1,1'-Biphenyl]-2,4-diamin-Derivat der Formel (I) oder dessen physiologisch verträgliches Salz enthält, 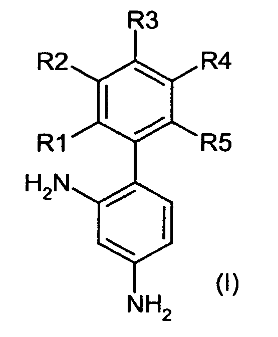 worin
**R1,R2,R3,R4,R5** gleich oder verschieden sein können und unabhängig voneinander Wasserstoff, ein Halogenatom (Fluor, Chlor, Brom, Jod), eine Cyanogruppe, eine Hydroxygruppe, eine C₁-C₄-Alkoxygruppe, eine C₁-C₄-Hydroxyalkoxygruppe, eine C₁-C₆-Alkylgruppe, eine C₁-C₄-Alkylthioethergruppe, eine Mercaptogruppe, eine Nitrogruppe, eine Aminogruppe (NH2), eine C₁-C₄-Monoalkylaminogruppe, eine Di(C₁-C₄-)-alkylaminogruppe, eine Trifluormethangruppe, eine -C(O)H-Gruppe, eine -C(O)CH₃-Gruppe, eine -C(O)CF₃-Gruppe, eine -Si(CH₃)₃-Gruppe, eine C₁-C₄-Hydroxyalkylgruppe, eine C₂-C₄ Dihydroxyalkylgruppe, eine -CH=CHR6-Gruppe, eine -(CH₂)ₚ-CO₂R7-Gruppe oder eine -(CH₂)ₚ-R8-Gruppe mit p= 1,2,3 oder 4, eine -C(R9)=NR10-Gruppe oder eine C(R12)H-NR13R14-Gruppe bedeuten, oder zwei nebeneinanderliegende Reste R1 bis R5 eine -O-CH2-O-Brücke bilden;
**R6** gleich Wasserstoff, einer Nitrogruppe, einer CO₂R7-Gruppe oder einer -C(O)CH₃-Gruppe ist;
**R7, R9 und R12** gleich oder verschieden sein können und unabhängig voneinander gleich Wasserstoff oder einer C₁-C₄-Alkylgruppe sind; **R8** gleich einer Aminogruppe oder einer Nitrilgruppe ist;
**R10, R13 und R14** gleich oder verschieden sein können und unabhängig voneinander gleich Wasserstoff, einer Hydroxygruppe, einer C₁-C₄-Alkylgruppe, einer C₁-C₄-Hydroxyalkylgruppe, einer C₂-C₄-Dihydroxyalkylgruppe oder einem Rest der Formel 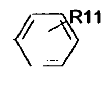 sind;
**R11** gleich Wasserstoff, einer Aminogruppe oder einer Hydroxygruppe ist.

2. Mittel nach Anspruch 1, **dadurch gekennzeichnet, dass** mindestens drei der Reste **R1** bis **R5** gleichWasserstoff sind.

3. Mittel nach einem der Ansprüche 1 und 2, **dadurch gekennzeichnet, dass** 3 der Reste **R1** bis **R5** gleich Wasserstoff sind und die beiden verbleibenden Reste unabhängig voneinander gleich Wasserstoff, einer Methoxygruppe, einer Hydroxygruppe, einer C₁-C₄-Alkylgruppe, einer C₁-C₄-Hydroxyalkylgruppe oder einer Aminogruppe sind, oder -wenn die beiden verbleibenden Reste nebeneinanderliegen- gemeinsam eine -O-CH2-O-Brücke bilden.

4. Mittel nach einem der Ansprüche 1 bis 3, **dadurch gekennzeichnet, dass** das [1,1'-Biphenyl]-2,4-diamin-Derivat der Formel (I) ausgewählt ist aus Biphenyl-2,4-diamin; 4-Benzo[1,3]dioxol-5-yl-benzol-1,3-diamin, 4'-Hydroxy-biphenyl-2,4-diamin; 4'-Amino-biphenyl-2,4-diamin; 3'-Hydroxy-biphenyl-2,4-diamin und 4'-Methoxy-biphenyl-2,4-diamin sowie deren physiologisch verträglichen Salzen.

5. Mittel nach einem der Ansprüche 1 bis 4, **dadurch gekennzeichnet, dass** das [1,1'-Biphenyl]-2,4-diamin der Formel (I) in einer Menge von 0,005 bis 20 Gewichtsprozent enthalten ist.

6. Mittel nach einem der Ansprüche 1 bis 5, **dadurch gekennzeichnet, dass** es einen pH-Wert von 6,5 bis 11,5 aufweist.

7. Mittel nach einem der Ansprüche 1 bis 6, **dadurch gekennzeichnet, dass** die Entwicklersubstanz ausgewählt ist aus der Gruppe bestehend aus 1,4-Diamino-benzol, 1,4-Diamino-2-methyl-benzol, 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methylanilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyethyl)-benzol, 1-(2,5-Diamino-phenyl)-ethanol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methylphenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1 H-pyrazol, 4,5-Diamino-1-[(4-methylphenyl)methyl]-1 H-pyrazol, 1-[(4-Chlorphenyl)methyl]-4,5-diamino-1H-pyrazol, 4,5-Diamino-1-methyl-1 H-pyrazol, 4,5-Diamino-3-methyl-1-methyl-1 H-pyrazol, 4,5-Diamino-3-methyl-1-methyl-1 H-pyrazol, 2-Amino-phenol, 2-Amino-6-methyl-phenol und 2-Amino-5-methyl-phenol.

8. Mittel nach einem der Ansprüche 1 bis 7, **dadurch gekennzeichnet, dass** die Entwicklersubstanzen und Kupplersubstanzen, bezogen auf die Gesamtmenge des Färbemittels, jeweils in einer Gesamtmenge von 0,005 bis 20 Gewichtsprozent enthalten sind.

9. Mittel nach einem der Ansprüche 1 bis 8, **dadurch gekennzeichnet, dass** es zusätzlich mindestens einen direktziehenden Farbstoff enthält.

10. Mittel nach einem der Ansprüche 1 bis 9, **dadurch gekennzeichnet, dass** es ein Haarfärbemittel ist.

11. [1,1'-Biphenyl]-2,4-diamin-Derivat, welches ausgewählt ist aus der Gruppe bestehend aus 4-Benzo[1,3]dioxol-5-yl-benzol-1,3-diamin; 2'-(2-Hydroxyethyl)-biphenyl-2,4-diamin; 2'-Acetyl-biphenyl-2,4-diamin; 2'-Amino-biphenyl-2,4-diamin; 2'-Brom-biphenyl-2,4-diamin; 2'-Chlor-biphenyl-2,4-diamin; 2'-Cyano-biphenyl-2,4-diamin; 2'-Ethyl-biphenyl-2,4-diamin; 2'-Fluor-biphenyl-2,4-diamin; 2'-Hydroxy-biphenyl-2,4-diamin; 2'-Methoxy-biphenyl-2,4-diamin; 2'-Methyl-biphenyl-2,4-diamin; 2'-Methylsulfanyl-biphenyl-2,4-diamin; 2'-Nitro-biphenyl-2,4-diamin; 2'-Trifluormethyl-biphenyl-2,4-diamin; 3'-(2-Hydroxyethyl)-biphenyl-2,4-diamin; 3'-Acetyl-biphenyl-2,4-diamin; 3'-Amino-biphenyl-2,4-diamin; 3'-Brom-biphenyl-2,4-diamin; 3'-Chlor-biphenyl-2,4-diamin; 3'-Cyano-biphenyl-2,4-diamin; 3'-Ethyl-biphenyl-2,4-diamin; 3'-Fluor-biphenyl-2,4-diamin; 3'-Hydroxy-biphenyl-2,4-diamin; 3'-Methoxy-biphenyl-2,4-diamin; 3'-Methyl-biphenyl-2,4-diamin; 3'-Methylsulfanyl-biphenyl-2,4-diamin; 3'-Nitro-biphenyl-2,4-diamin; 3'-Trifluormethyl-biphenyl-2,4-diamin; 4'-(2-Hydroxyethyl)-biphenyl-2,4-diamin; 4'-Acetyl-biphenyl-2,4-diamin; 4'-Amino-biphenyl-2,4-diamin; 4'-Brom-biphenyl-2,4-diamin; 4'-Chlor-biphenyl-2,4-diamin; 4'-Cyano-biphenyl-2,4-diamin; 4'-Ethyl-biphenyl-2,4-diamin; 4'-Fluor-biphenyl-2,4-diamin; 4'-Hydroxy-biphenyl-2,4-diamin; 4'-Methoxy-biphenyl-2,4-diamin; 4'-Methyl-biphenyl-2,4-diamin; 4'-Methylsulfanyl-biphenyl-2,4-diamin; 4'-Nitro-biphenyl-2,4-diamin; 4'-Trifluormethyl-biphenyl-2,4-diamin; 2'-Amino-3'-amino-biphenyl-2,4-diamin; 2'-Amino-3'-hydroxy-biphenyl-2,4-diamin; 2'-Amino-3'-methoxy-biphenyl-2,4-diamin; 2'-Amino-3'-methyl-biphenyl-2,4-diamin; 2'-Amino-4'-amino-biphenyl-2,4-diamin; 2'-Amino-4'-hydroxy-biphenyl-2,4-diamin; 2'-Amino-4'-methoxy-biphenyl-2,4-diamin; 2'-Amino-4'-methyl-biphenyl-2,4-diamin; 2'-Amino-5'-amino-biphenyl-2,4-diamin; 2'-Amino-5'-hydroxybiphenyl-2,4-diamin; 2'-Amino-5'-methoxy-biphenyl-2,4-diamin; 2'-Amino-5'-methyl-biphenyl-2,4-diamin; 2'-Amino-6'-amino-biphenyl-2,4-diamin; 2'-Amino-6'-hydroxy-biphenyl-2,4-diamin; 2'-Amino-6'-methoxy-biphenyl-2,4-diamin; 2'-Amino-6'-methyl-biphenyl-2,4-diamin; 2'-Hydroxy-3'-aminobiphenyl-2,4-diamin; 2'-Hydroxy-3'-hydroxy-biphenyl-2,4-diamin; 2'-Hydroxy-3'-methoxy-biphenyl-2,4-diamin; 2'-Hydroxy-3'-methyl-biphenyl-2,4-diamin; 2'-Hydroxy-4'-amino-biphenyl-2,4-diamin; 2'-Hydroxy-4'-hydroxy-biphenyl-2,4-diamin; 2'-Hydroxy-4'-methoxy-biphenyl-2,4-diamin; 2'-Hydroxy-4'-methyl-biphenyl-2,4-diamin; 2'-Hydroxy-5'-amino-biphenyl-2,4-diamin; 2'-Hydroxy-5'-hydroxy-biphenyl-2,4-diamin; 2'-Hydroxy-5'-methoxy-biphenyl-2,4-diamin; 2'-Hydroxy-5'-methyl-biphenyl-2,4-diamin; 2'-Hydroxy-6'-amino-biphenyl-2,4-diamin; 2'-Hydroxy-6'-hydroxy-biphenyl-2,4-diamin; 2'-Hydroxy-6'-methoxy-biphenyl-2,4-diamin; 2'-Hydroxy-6'-methyl-biphenyl-2,4-diamin; 2'-Methoxy-3'-amino-biphenyl-2,4-diamin; 2'-Methoxy-3'-hydroxy-biphenyl-2,4-diamin; 2'-Methoxy-3'-methoxy-biphenyl-2,4-diamin; 2'-Methoxy-3'-methyl-biphenyl-2,4-diamin; 2'-Methoxy-4'-amino-biphenyl-2,4-diamin; 2'-Methoxy-4'-hydroxy-biphenyl-2,4-diamin; 2'-Methoxy-4'-methoxy-biphenyl-2,4-diamin; 2'-Methoxy-4'-methyl-biphenyl-2,4-diamin; 2'-Methoxy-5'-amino-biphenyl-2,4-diamin; 2'-Methoxy-5'-hydroxy-biphenyl-2,4-diamin; 2'-Methoxy-5'-methoxy-biphenyl-2,4-diamin; 2'-Methoxy-5'-methyl-biphenyl-2,4-diamin; 2'-Methoxy-6'-amino-biphenyl-2,4-diamin; 2'-Methoxy-6'-hydroxy-biphenyl-2,4-diamin; 2'-Methoxy-6'-methoxy-biphenyl-2,4-diamin; 2'-Methoxy-6'-methyl-biphenyl-2,4-diamin; 2'-Methyl-3'-amino-biphenyl-2,4-diamin; 2'-Methyl-3'-hydroxy-biphenyl-2,4-diamin; 2'-Methyl-3'-methoxy-biphenyl-2,4-diamin; 2'-Methyl-3'-methyl-biphenyl-2,4-diamin; 2'-Methyl-4'-amino-biphenyl-2,4-diamin; 2'-Methyl-4'-hydroxybiphenyl-2,4-diamin; 2'-Methyl-4'-methoxy-biphenyl-2,4-diamin; 2'-Methyl-4'-methyl-biphenyl-2,4-diamin; 2'-Methyl-5'-amino-biphenyl-2,4-diamin; 2'-Methyl-5'-hydroxy-biphenyl-2,4-diamin; 2'-Methyl-5'-methoxy-biphenyl-2,4-diamin; 2'-Methyl-5'-methyl-biphenyl-2,4-diamin; 2'-Methyl-6'-aminobiphenyl-2,4-diamin; 2'-Methyl-6'-hydroxy-biphenyl-2,4-diamin; 2'-Methyl-6'-methoxy-biphenyl-2,4-diamin; 2'-Methyl-6'-methyl-biphenyl-2,4-diamin; 3'-Amino-4'-hydroxy-biphenyl-2,4-diamin; 3'-Amino-4'-methoxy-biphenyl-2,4-diamin; 3'-Amino-4'-methyl-biphenyl-2,4-diamin; 3'-Amino-5'-aminobiphenyl-2,4-diamin; 3'-Amino-5'-hydroxy-biphenyl-2,4-diamin; 3'-Amino-5'-methoxy-biphenyl-2,4-diamin; 3'-Amino-5'-methyl-biphenyl-2,4-diamin; 3'-Hydroxy-4'-amino-biphenyl-2,4-diamin; 3'-Hydroxy-4'-hydroxy-biphenyl-2,4-diamin; 3'-Hydroxy-4'-methoxy-biphenyl-2,4-diamin; 3'-Hydroxy-4'-methyl-biphenyl-2,4-diamin; 3'-Hydroxy-5'-amino-biphenyl-2,4-diamin; 3'-Hydroxy-5'-hydroxy-biphenyl-2,4-diamin; 3'-Hydroxy-5'-methoxy-biphenyl-2,4-diamin; 3'-Hydroxy-5'-methyl-biphenyl-2,4-diamin; 3'-Methoxy-4'-amino-biphenyl-2,4-diamin; 3'-Methoxy-4'-hydroxy-biphenyl-2,4-diamin; 3'-Methoxy-4'-methoxy-biphenyl-2,4-diamin; 3'-Methoxy-4'-methyl-biphenyl-2,4-diamin; 3'-Methoxy-5'-amino-biphenyl-2,4-diamin; 3'-Methoxy-5'-hydroxy-biphenyl-2,4-diamin; 3'-Methoxy-5'-methoxy-biphenyl-2,4-diamin; 3'-Methoxy-5'-methyl-biphenyl-2,4-diamin; 3'-Methyl-4'-amino-biphenyl-2,4-diamin; 3'-Methyl-4'-hydroxy-biphenyl-2,4-diamin; 3'-Methyl-4'-methoxy-biphenyl-2,4-diamin; 3'-Methyl-4'-methyl-biphenyl-2,4-diamin; 3'-Methyl-5'-amino-biphenyl-2,4-diamin; 3'-Methyl-5'-hydroxybiphenyl-2,4-diamin; 3'-Methyl-5'-methoxy-biphenyl-2,4-diamin und 3'-Methyl-5'-methyl-biphenyl-2,4-diamin, sowie deren physiologisch verträglichen Salzen.

## Claims

1. Agent for the oxidative colouring of keratin fibres on the basis of a developer substance-coupler substance combination, **characterized in that** it comprises, as coupler substance, at least one [1,1'-biphenyl]-2,4-diamine derivative of the formula (I) or physiologically compatible salt thereof, 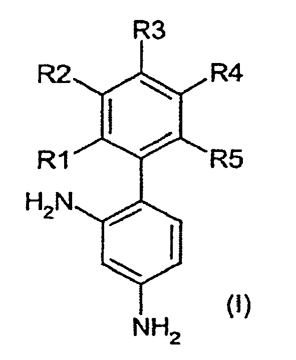 in which
**R1,R2,R3,R4,R5** may be identical or different and, independently of one another, are hydrogen, a halogen atom (fluorine, chlorine, bromine, iodine), a cyano group, a hydroxy group, a C₁-C₄-alkoxy group, a C₁-C₄-hydroxyalkoxy group, a C₁-C₆-alkyl group, a C₁-C₄-alkylthio ether group, a mercapto group, a nitro group, an amino group (NH2), a C₁-C₄-monoalkylamino group, a di(C₁-C₄)-alkylamino group, a trifluoromethane group, a -C(O)H group, a -C(O)CH₃ group, a -C(O)CF₃ group, an -Si(CH₃)₃ group, a C₁-C₄-hydroxyalkyl group, a C₂-C₄-dihydroxyalkyl group, a -CH=CHR6 group, a -(CH₂)ₚ-CO₂R7 group or a -(CH₂)ₚ-R8 group where p = 1, 2, 3 or 4, a -C(R9)=NR10 group or a C(R12)H-NR13R14 group, or two adjacent radicals R1 to R5 form a -O-CH2-O bridge;
**R6** is hydrogen, a nitro group, a CO₂R7 group or a -C(O)CH₃ group;
**R7,R9 and R12 m**ay be identical or different and, independently of one another, are hydrogen or a C₁-C₄-alkyl group;
**R8** is an amino group or a nitrile group;
**R10,R13 and R14** may be identical or different and, independently of one another, are hydrogen, a hydroxy group, a C₁-C₄-alkyl group, a C₁-C₄-hydroxyalkyl group, a C₂-C₄-dihydroxyalkyl group or a radical of the formula 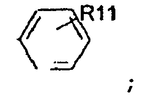
**R11** is hydrogen, an amino group or a hydroxy group.

2. Agent according to Claim 1, **characterized in that** at least three of the radicals **R1** to **R5** are hydrogen.

3. Agent according to one of Claims 1 and 2,
**characterized in that** 3 of the radicals **R1** to **R5** are hydrogen and the two remaining radicals, independently of one another, are hydrogen, a methoxy group, a hydroxy group, a C₁-C₄-alkyl group, a C₁-C₄-hydroxyalkyl group or an amino group, or - if the two remaining radicals are adjacent - together form a -O-CH₂-O bridge.

4. Agent according to one of Claims 1 to 3, **characterized in that** the [1,1'-biphenyl]-2,4-diamine derivative of the formula (I) is chosen from biphenyl-2,4-diamine; 4-benzo[1,3]dioxol-5-ylbenzene-1,3-diamine, 4'-hydroxybiphenyl-2,4-diamine; 4'-aminobiphenyl-2,4-diamine; 3'-hydroxybiphenyl-2,4-diamine and 4'-methoxybiphenyl-2,4-diamine, and physiologically compatible salts thereof.

5. Agent according to one of Claims 1 to 4, **characterized in that** the [1,1'-biphenyl]-2,4-diamine of the formula (I) is present in an amount of from 0.005 to 20 per cent by weight.

6. Agent according to one of Claims 1 to 5, **characterized in that** it has a pH of from 6.5 to 11.5.

7. Agent according to one of Claims 1 to 6, **characterized in that** the developer substance is chosen from the group consisting of 1,4-diaminobenzene, 1,4-diamino-2-methylbenzene, 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(thiophen-2-yl)benzene, 1,4-diamino-2-(thiophen-3-yl)benzene, 1,4-diamino-2-(pyridin-3-yl)benzene, 2,5-diaminobiphenyl, 1,4-diamino-2-methoxymethylbenzene, 1,4-diamino-2-aminomethylbenzene, 1,4-diamino-2-hydroxymethylbenzene, 1,4-diamino-2-(2-hydroxyethoxy)-benzene, 2-(2-(acetylamino)ethoxy)-1,4-diaminobenzene, 4-phenylaminoaniline, 4-dimethylaminoaniline, 4-diethylaminoaniline, 4-dipropylaminoaniline, 4- [ethyl (2-hydroxyethyl) amino] aniline, 4- [di (2-hydroxyethyl) amino] aniline, 4- [di (2-hydroxyethyl) amino] -2-methylaniline, 4-[(2-methoxyethyl)amino]aniline, 4-[(3-hydroxypropyl)amino]aniline, 4-[(2,3-dihydroxypropyl)-amino]aniline, 1,4-diamino-2-(2-hydroxyethyl)benzene, 1-(2,5-diaminophenyl)ethanol, 1,4-diamino-2-(1-methylethyl)benzene, 1,3-bis[(4-aminophenyl)(2-hydroxyethyl)-amino]-2-propanol, 1,4-bis[(4-aminophenyl)amino]butane, 1,8-bis(2,5-diaminophenoxy)-3,6-dioxaoctane, 4-aminophenol, 4-amino-3-methylphenol, 4-amino-3-(hydroxymethyl)phenol, 4-amino-3-fluorophenol, 4-methylamino-phenol, 4-amino-2-(aminomethyl)phenol, 4-amino-2-(hydroxymethyl)phenol, 4-amino-2-fluorophenol, 4-amino-2-[(2-hydroxyethyl)amino]methylphenol, 4-amino-2-methylphenol, 4-amino-2-(methoxymethyl)phenol, 4-amino-2-(2-hydroxyethyl)phenol, 5-aminosalicylic acid, 2,5-diaminopyridine, 2,4,5,6-tetraaminopyrimidine, 2,5,6-triamino-4-(1H)-pyrimidone, 4,5-diamino-1-(2-hydroxyethyl)-1H-pyrazole, 4,5-diamino-1-(1-methylethyl)-1H-pyrazole, 4,5-diamino-1-[(4-methylphenyl)-methyl]-1H-pyrazole, 1-[(4-chlorophenyl)methyl]-4,5-diamino-1H-pyrazole, 4,5-diamino-1-methyl-1H-pyrazole, 4,5-diamino-3-methyl-1-methyl-1H-pyrazole, 4,5-diamino-3-methyl-1-methyl-1H-pyrazole, 2-aminophenol, 2-amino-6-methylphenol and 2-amino-5-methylphenol.

8. Agent according to one of Claims 1 to 7, **characterized in that** the developer substances and coupler substances, based on the total amount of the colouring agent, are in each case present in a total amount of from 0.005 to 20 per cent by weight.

9. Agent according to one of Claims 1 to 8, **characterized in that** it additionally comprises at least one direct dye.

10. Agent according to one of Claims 1 to 9, **characterized in that** it is a hair colouring agent.

11. [1,1'-Biphenyl]-2,4-diamine derivative which is chosen from the group consisting of 4-benzo [1,3]dioxol-5-ylbenzene-1,3-diamine; 2'-(2-hydroxyethyl)biphenyl-2,4-diamine; 2'-acetylbiphenyl-2,4-diamine; 2'-aminobiphenyl-2,4-diamine; 2'-bromobiphenyl-2,4-diamine; 2'-chlorobiphenyl-2,4-diamine; 2'-cyanobiphenyl-2,4-diamine; 2'-ethylbiphenyl-2,4-diamine; 2'-fluorobiphenyl-2,4-diamine; 2'-hydroxybiphenyl-2,4-diamine; 2'-methoxybiphenyl-2,4-diamine; 2'-methylbiphenyl-2,4-diamine; 2'-methylsulphanylbiphenyl-2,4-diamine; 2'-nitrobiphenyl-2,4-diamine; 2'-trifluoromethylbiphenyl-2,4-diamine; 3'-(2-hydroxyethyl)biphenyl-2,4-diamine; 3'-acetylbiphenyl-2,4-diamine; 3'-aminobiphenyl-2,4-diamine; 3'-bromobiphenyl-2,4-diamine; 3'-chlorobiphenyl-2,4-diamine; 3'-cyanobiphenyl-2,4-diamine; 3'-ethylbiphenyl-2,4-diamine; 3'-fluorobiphenyl-2,4-diamine; 3'-hydroxybiphenyl-2,4-diamine; 3'-methoxybiphenyl-2,4-diamine; 3'-methylbiphenyl-2,4-diamine; 3'-methylsulphanylbiphenyl-2,4-diamine; 3'-nitrobiphenyl-2,4-diamine; 3'-trifluoromethylbiphenyl-2,4-diamine; 4'-(2-hydroxyethyl)biphenyl-2,4-diamine; 4'-acetylbiphenyl-2,4-diamine; 4'-aminobiphenyl-2,4-diamine; 4'-bromobiphenyl-2,4-diamine; 4'-chlorobiphenyl-2,4-diamine; 4'-cyanobiphenyl-2,4-diamine; 4'-ethylbiphenyl-2,4-diamine; 4'-fluorobiphenyl-2,4-diamine; 4'-hydroxybiphenyl-2,4-diamine; 4'-methoxybiphenyl-2,4-diamine; 4'-methylbiphenyl-2,4-diamine; 4'-methylsulphanylbiphenyl-2,4-diamine; 4'-nitrobiphenyl-2,4-diamine; 4'-trifluoromethylbiphenyl-2,4-diamine; 2'-amino-3'-aminobiphenyl-2,4-diamine; 2'-amino-3'-hydroxybiphenyl-2,4-diamine; 2'-amino-3'-methoxybiphenyl-2,4-diamine; 2'-amino-3'-methylbiphenyl-2,4-diamine; 2'-amino-4'-aminobiphenyl-2,4-diamine; 2'-amino-4'-hydroxybiphenyl-2,4-diamine; 2'-amino-4'-methoxybiphenyl-2,4-diamine; 2'-amino-4'-methylbiphenyl-2,4-diamine; 2'-amino-5'-aminobiphenyl-2,4-diamine; 2'-amino-5'-hydroxybiphenyl-2,4-diamine; 2'-amino-5'-methoxybiphenyl-2,4-diamine; 2'-amino-5'-methylbiphenyl-2,4-diamine; 2'-amino-6'-aminobiphenyl-2,4-diamine; 2'-amino-6'-hydroxybiphenyl-2,4-diamine; 2'-amino-6'-methoxybiphenyl-2,4-diamine; 2'-amino-6'-methylbiphenyl-2,4-diamine; 2'-hydroxy-3'-aminobiphenyl-2,4-diamine; 2'-hydroxy-3'-hydroxybiphenyl-2,4-diamine; 2'-hydroxy-3'-methoxybiphenyl-2,4-diamine; 2'-hydroxy-3'-methylbiphenyl-2,4-diamine; 2'-hydroxy-4'-aminobiphenyl-2,4-diamine; 2'-hydroxy-4'-hydroxybiphenyl-2,4-diamine; 2'-hydroxy-4'-methoxybiphenyl-2,4-diamine; 2'-hydroxy-4'-methylbiphenyl-2,4-diamine; 2'-hydroxy-5'-aminobiphenyl-2,4-diamine; 2'-hydroxy-5'-hydroxybiphenyl-2,4-diamine; 2'-hydroxy-5'-methoxybiphenyl-2,4-diamine; 2'-hydroxy-5'-methylbiphenyl-2,4-diamine; 2'-hydroxy-6'-aminobiphenyl-2,4-diamine; 2'-hydroxy-6'-hydroxybiphenyl-2,4-diamine; 2'-hydroxy-6'-methoxybiphenyl-2,4-diamine; 2'-hydroxy-6'-methylbiphenyl-2,4-diamine; 2'-methoxy-3'-aminobiphenyl-2,4-diamine; 2'-methoxy-3'-hydroxybiphenyl-2,4-diamine; 2'-methoxy-3'-methoxybiphenyl-2,4-diamine; 2'-methoxy-3'-methylbiphenyl-2,4-diamine; 2'-methoxy-4'-aminobiphenyl-2,4-diamine; 2'-methoxy-4'-hydroxybiphenyl-2,4-diamine; 2'-methoxy-4'-methoxybiphenyl-2,4-diamine; 2'-methoxy-4'-methylbiphenyl-2,4-diamine; 2'-methoxy-5'-aminobiphenyl-2,4-diamine; 2'-methoxy-5'-hydroxybiphenyl-2,4-diamine; 2'-methoxy-5'-methoxybiphenyl-2,4-diamine; 2'-methoxy-5'-methylbiphenyl-2,4-diamine; 2'-methoxy-6'-aminobiphenyl-2,4-diamine; 2'-methoxy-6'-hydroxybiphenyl-2,4-diamine; 2'-methoxy-6'-methoxybiphenyl-2,4-diamine; 2'-methoxy-6'-methylbiphenyl-2,4-diamine; 2'-methyl-3'-aminobiphenyl-2,4-diamine; 2'-methyl-3'-hydroxybiphenyl-2,4-diamine; 2'-methyl-3'-methoxybiphenyl-2,4-diamine; 2'-methyl-3'-methylbiphenyl-2,4-diamine; 2'-methyl-4'-aminobiphenyl-2,4-diamine; 2'-methyl-4'-hydroxybiphenyl-2,4-diamine; 2'-methyl-4'-methoxybiphenyl-2,4-diamine; 2'-methyl-4'-methylbiphenyl-2,4-diamine; 2'-methyl-5'-aminobiphenyl-2,4-diamine; 2'-methyl-5'-hydroxybiphenyl-2,4-diamine; 2'-methyl-5'-methoxybiphenyl-2,4-diamine; 2'-methyl-5'-methylbiphenyl-2,4-diamine; 2'-methyl-6'-aminobiphenyl-2,4-diamine; 2'-methyl-6'-hydroxybiphenyl-2,4-diamine; 2'-methyl-6'-methoxybiphenyl-2,4-diamine; 2'-methyl-6'-methylbiphenyl-2,4-diamine; 3'-amino-4'-hydroxybiphenyl-2,4-diamine; 3'-amino-4'-methoxybiphenyl-2,4-diamine; 3'-amino-4'-methylbiphenyl-2,4-diamine; 3'-amino-5'-aminobiphenyl-2,4-diamine; 3'-amino-5'-hydroxybiphenyl-2,4-diamine; 3'-amino-5'-methoxybiphenyl-2,4-diamine; 3'-amino-5'-methylbiphenyl-2,4-diamine; 3'-hydroxy-4'-aminobiphenyl-2,4-diamine; 3'-hydroxy-4'-hydroxybiphenyl-2,4-diamine; 3'-hydroxy-4'-methoxybiphenyl-2,4-diamine; 3'-hydroxy-4'-methylbiphenyl-2,4-diamine; 3'-hydroxy-5'-aminobiphenyl-2,4-diamine; 3'-hydroxy-5'-hydroxybiphenyl-2,4-diamine; 3'-hydroxy-5'-methoxybiphenyl-2,4-diamine; 3'-hydroxy-5'-methylbiphenyl-2,4-diamine; 3'-methoxy-4'-aminobiphenyl-2,4-diamine; 3'-methoxy-4'-hydroxybiphenyl-2,4-diamine; 3'-methoxy-4'-methoxybiphenyl-2,4-diamine; 3'-methoxy-4'-methylbiphenyl-2,4-diamine; 3'-methoxy-5'-aminobiphenyl-2,4-diamine; 3'-methoxy-5'-hydroxybiphenyl-2,4-diamine; 3'-methoxy-5'-methoxybiphenyl-2,4-diamine; 3'-methoxy-5'-methylbiphenyl-2,4-diamine; 3'-methyl-4'-aminobiphenyl-2,4-diamine; 3'-methyl-4'-hydroxybiphenyl-2,4-diamine; 3'-methyl-4'-methoxybiphenyl-2,4-diamine; 3'-methyl-4'-methylbiphenyl-2,4-diamine; 3'-methyl-5'-aminobiphenyl-2,4-diamine; 3'-methyl-5'-hydroxybiphenyl-2,4-diamine; 3'-methyl-5'-methoxybiphenyl-2,4-diamine and 3'-methyl-5'-methylbiphenyl-2,4-diamine and physiologically compatible salts thereof.

## Revendications

1. Composition pour la teinture oxydative de fibres kératiniques à base d'une combinaison de substance de révélation/substance de couplage, **caractérisée en ce qu'**elle contient, en tant que substance de couplage, au moins un dérivé de [1,1'-biphényl]-2,4-diamine de formule (I) ou son sel physiologiquement acceptable, 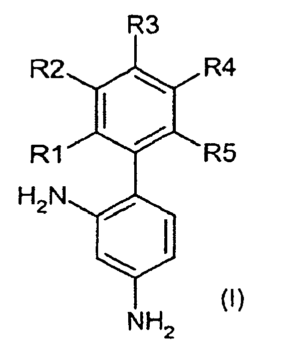 dans laquelle
**R1,R2,R3,R4,R5** peuvent être identiques ou différents et représentent, indépendamment les uns des autres, un atome d'hydrogène, un atome d'halogène (un atome de fluore, un atome de chlore, un atome de brome, un atome d'iode), un groupe cyano, un groupe hydroxy, un groupe alcoxy en C₁-C₄, un groupe hydroxyalcoxy en C₁-C₄, un groupe alkyle en C₁-C₆, un groupe C₁-C₄-alkylthioéther, un groupe mercapto, un groupe nitro, un groupe amino (NH2), un groupe monoalkylamino en C₁-C₄, un groupe di-(C₁-C₄)-alkylamino, un groupe trifluorométhane, un groupe -C (O) H, un groupe -C(O)CH₃, un groupe -C(O)CF₃, un groupe -Si(CH₃)₃, un groupe hydroxyalkyle en C₁-C₄, un groupe C₂-C₄-dihydroxyalkyle, un groupe -CH=CHR6, un groupe -(CH₂)ₚ-CO₂R7 ou un groupe - (CH2)p-R8 dans lesquels p vaut 1, 2, 3 ou 4, un groupe -C(R9)=NR10 ou un groupe C(R12)H-NR13R14, ou deux radicaux adjacents R1 à R5 forment un pont -O-CH2-O ;
**R6** représente un atome d'hydrogène, un groupe nitro, un groupe CO₂R7 ou un groupe -C(O)CH₃ ;
**R7,R9 et R12** peuvent être identiques ou différents et représentent, indépendamment les uns des autres, un atome d'hydrogène ou un groupe alkyle en C₁-C₄ ;
**R8** représente un groupe amino ou un groupe nitrile ;
**R10,R13 et R14** peuvent être identiques ou différents et représentent, indépendamment les uns des autres, un atome d'hydrogène, un groupe hydroxy, un groupe alkyle en C₁-C₄, un groupe hydroxyalkyle en C₁-C₄, un groupe C₂-C₄-dihydroxyalkyle ou un radical de formule 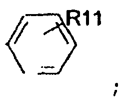
**R11** représente un atome d'hydrogène, un groupe amino ou un groupe hydroxy.

2. Composition selon la revendication 1, **caractérisée en ce qu'**au moins trois des radicaux **R1** à **R5** représentent un atome d'hydrogène.

3. Composition selon l'une quelconque des revendications 1 et 2, **caractérisée en ce que** 3 des radicaux R1 à R5 représentent un atome d'hydrogène et les deux radicaux restants représentent, indépendamment l'un de l'autre, un atome d'hydrogène, un groupe méthoxy, un groupe hydroxy, un groupe alkyle en C₁-C₄, un groupe hydroxyalkyle en C₁-C₄ ou un groupe amino, ou - si les deux radicaux restants sont adjacents forment conjointement un pont -O-CH₂-O.

4. Composition selon l'une quelconque des revendications 1 à 3, **caractérisée en ce que** le dérivé de [1,1'-biphényl]-2,4-diamine de formule (I) est choisi parmi la biphényl-2,4-diamine ; la 4-benzo[1,3]dioxol-5-ylbenzène-1,3-diamine, la 4'-hydroxybiphényl-2,4-diamine ; la 4'-aminobiphényl-2,4-diamine ; la 3'-hydroxybiphényl-2,4-diamine et la 4'-méthoxybiphényl-2,4-diamine, ainsi que leurs sels physiologiquement acceptables.

5. Composition selon l'une quelconque des revendications 1 à 4, **caractérisée en ce que** la [1,1'-biphényl]-2,4-diamine de formule (I) est présente en une quantité de 0,005 à 20 pour cent en poids.

6. Composition selon l'une quelconque des revendications 1 à 5, **caractérisée en ce qu'**elle présente un pH de 6,5 à 11,5.

7. Composition selon l'une quelconque des revendications 1 à 6, **caractérisée en ce que** la substance de révélation est choisie parmi le groupe constitué du 1,4-diaminobenzène, du 1,4-diamino-2-méthylbenzène, du 1,4-diamino-2,6-diméthylbenzène, du 1,4-diamino-3,5-diéthylbenzène, du 1,4-diamino-2,5-diméthylbenzène, du 1,4-diamino-2,3-diméthylbenzène, du 2-chloro-1,4-diaminobenzène, du 1,4-diamino-2-(thiophen-2-yl)benzène, du 1,4-diamino-2-(thiophén-3-yl)benzène, du 1,4-diamino-2-(pyridin-3-yl)benzène, du 2,5-diaminobiphényle, du 1,4-diamino-2-méthoxyméthylbenzène, du 1,4-diamino-2-aminométhylbenzène, du 1,4-diamino-2-hydroxyméthylbenzène, du 1,4-diamino-2-(2-hydroxyéthoxy)benzène, du 2-(2-(acétylamino)éthoxy)-1,4-diaminobenzène, de la 4-phénylaminoaniline, de la 4-diméthylaminoaniline, de la 4-diéthylaminoaniline, de la 4-dipropylaminoaniline, de la 4-[éthyl(2-hydroxyéthyl)amino]aniline, de la 4-[di(2-hydroxyéthyl)amino]aniline, de la 4-[di(2-hydroxyéthyl)amino]-2-méthylaniline, de la 4-[(2-méthoxyéthyl)amino]aniline, de la 4-[(3-hydroxypropyl)amino]aniline, de la 4-[(2,3-dihydroxypropyl)amino]aniline, du 1,4-diamino-2-(2-hydroxyéthyl)benzène, du 1-(2,5-diaminophényl)éthanol, du 1,4-diamino-2-(1-méthyléthyl)benzène, du 1,3-bis[(4-aminophényl)(2-hydroxyéthyl)amino]-2-propanol, du 1,4-bis[(4-aminophényl)amino]butane, du 1,8-bis(2,5-diaminophénoxy)-3,6-dioxaoctane, du 4-aminophénol, du 4-amino-3-méthylphénol, du 4-amino-3-(hydroxyméthyl)phénol, du 4-amino-3-fluorophénol, du 4-méthylaminophénol, du 4-amino-2-(aminométhyl)phénol, du 4-amino-2-(hydroxyméthyl)phénol, du 4-amino-2-fluorophénol, du 4-amino-2-[(2-hydroxyéthyl)amino]méthylphénol, du 4-amino-2-méthylphénol, du 4-amino-2-(méthoxyméthyl)phénol, du 4-amino-2-(2-hydroxyéthyl)phénol, de l'acide 5-aminosalicylique, de la 2,5-diaminopyridine, de la 2,4,5,6-tétraaminopyrimidine, du 2,5,6-triamino-4-(1H)-pyrimidone, du 4,5-diamino-1-(2-hydroxyéthyl)-1H-pyrazole, du 4,5-diamino-1-(1-méthyléthyl)-1H-pyrazole, du 4,5-diamino-1-[(4-méthylphényl)méthyl]-1H-pyrazole, du 1-[(4-chlorophényl)méthyl]-4,5-diamino-1H-pyrazole, du 4,5-diamino-1-méthyl-1H-pyrazole, du 4,5-diamino-3-méthyl-1-méthyl-1H-pyrazole, du 4,5-diamino-3-méthyl-1-méthyl-1H-pyrazole, du 2-aminophénol, du 2-amino-6-méthylphénol et du 2-amino-5-méthylphénol.

8. Composition selon l'une quelconque des revendications 1 à 7, **caractérisée en ce que** les substances de révélation et les substances de couplage sont présentes dans chaque cas en une quantité totale de 0,005 à 20 pour cent en poids, par rapport à la quantité totale de la composition de teinture.

9. Composition selon l'une quelconque des revendications 1 à 8, **caractérisée en ce qu'**elle contient en outre au moins un colorant direct.

10. Composition selon l'une quelconque des revendications 1 à 9, **caractérisée en ce qu'**il s'agit d'une composition de teinture capillaire.

11. Dérivé de [1,1'-biphényl]-2,4-diamine, qui est choisi parmi le groupe constitué de la 4-benzo[1,3]dioxol-5-ylbenzène-1,3-diamine ; de la 2'-(2-hydroxyéthyl)biphényl-2,4-diamine ; de la 2'-acétylbiphényl-2,4-diamine ; de la 2'-aminobiphényl-2,4-diamine ; de la 2'-bromobiphényl-2,4-diamine ; de la 2'-chlorobiphényl-2,4-diamine ; de la 2'-cyanobiphényl-2,4-diamine ; de la 2'-éthylbiphényl-2,4-diamine ; de la 2'-fluorobiphényl-2,4-diamine ; de la 2'-hydroxybiphényl-2,4-diamine ; de la 2'-méthoxybiphényl-2,4-diamine ; de la 2'-méthylbiphényl-2,4-diamine ; de la 2'-méthylsulfanylbiphényl-2,4-diamine ; de la 2'-nitrobiphényl-2,4-diamine ; de la 2'-trifluorométhylbiphényl-2,4-diamine ; de la 3'-(2-hydroxyéthyl)biphényl-2,4-diamine ; de la 3'-acétylbiphényl-2,4-diamine ; de la 3'-aminobiphényl-2,4-diamine ; de la 3'-bromobiphényl-2,4-diamine ; de la 3'-chlorobiphényl-2,4-diamine ; de la 3'-cyanobiphényl-2,4-diamine ; de la 3'-éthylbiphényl-2,4-diamine ; de la 3'-fluorobiphényl-2,4-diamine ; de la 3'-hydroxybiphényl-2,4-diamine ; de la 3'-méthoxybiphényl-2,4-diamine ; de la 3'-méthylbiphényl-2,4-diamine ; de la 3'-méthylsulfanylbiphényl-2,4-diamine ; de la 3'-nitrobiphényl-2,4-diamine ; de la 3'-trifluorométhylbiphényl-2,4-diamine ; de la 4'-(2-hydroxyéthyl)biphényl-2,4-diamine ; de la 4'-acêtylbiphényl-2,4-diamine ; de la 4'-aminobiphényl-2,4-diamine ; de la 4'-bromobiphényl-2,4-diamine ; de la 4'-chlorobiphényl-2,4-diamine ; de la 4'-cyanobiphényl-2,4-diamine ; de la 4'-êthylbiphényl-2,4-diamine ; de la 4'-fluorobiphényl-2,4-diamine ; de la 4'-hydroxybiphényl-2,4-diamine ; de la 4'-méthoxybiphényl-2,4-diamine ; de la 4'-méthylbiphényl-2,4-diamine ; de la 4'-méthylsulfanylbiphényl-2,4-diamine ; de la 4'-nitrobiphényl-2,4-diamine ; de la 4'-trifluorométhylbiphényl-2,4-diamine ; de la 2'-amino-3'-aminobiphényl-2,4-diamine ; de la 2'-amino-3'-hydroxybiphényl-2,4-diamine ; de la 2'-amino-3'-méthoxybiphényl-2,4-diamine ; de la 2'-amino-3'-méthylbiphényl-2,4-diamine ; de la 2'-amino-4'-aminobiphényl-2,4-diamine ; de la 2'-amino-4'-hydroxybiphényl-2,4-diamine ; de la 2'-amino-4'-méthoxybiphényl-2,4-diamine ; de la 2'-amino-4'-méthylbiphényl-2,4-diamine ; de la 2'-amino-5'-aminobiphényl-2,4-diamine ; de la 2'-amino-5'-hydroxybiphényl-2,4-diamine ; de la 2'-amino-5'-méthoxybiphényl-2,4-diamine ; de la 2'-amino-5'-méthylbiphényl-2,4-diamine ; de la 2'-amino-6'-aminobiphényl-2,4-diamine ; de la 2'-amino-6'-hydroxybiphényl-2,4-diamine ; de la 2'-amino-6'-méthoxybiphényl-2,4-diamine ; de la 2'-amino-6'-méthylbiphényl-2,4-diamine ; de la 2'-hydroxy-3'-aminobiphényl-2,4-diamine ; de la 2'-hydroxy-3'-hydroxybiphényl-2,4-diamine ; de la 2'-hydroxy-3'-méthoxybiphényl-2,4-diamine ; de la 2'-hydroxy-3'-méthylbiphényl-2,4-diamine ; de la 2'-hydroxy-4'-aminobiphényl-2,4-diamine ; de la 2'-hydroxy-4'-hydroxybiphényl-2,4-diamine ; de la 2'-hydroxy-4'-méthoxybiphényl-2,4-diamine ; de la 2'-hydroxy-4'-méthylbiphényl-2,4-diamine ; de la 2'-hydroxy-5'-aminobiphényl-2,4-diamine ; de la 2'-hydroxy-5'-hydroxybiphényl-2,4-diamine ; de la 2'-hydroxy-5'-méthoxybiphényl-2,4-diamine ; de la 2'-hydroxy-5'-méthylbiphényl-2,4-diamine ; de la 2'-hydroxy-6'-aminobiphényl-2,4-diamine ; de la 2'-hydroxy-6'-hydroxybiphényl-2,4-diamine ; de la 2'-hydroxy-6'-méthoxybiphényl-2,4-diamine ; de la 2'-hydroxy-6'-méthylbiphényl-2,4-diamine ; de la 2'-méthoxy-3'-aminobiphényl-2,4-diamine ; de la 2'-méthoxy-3'-hydroxybiphényl-2,4-diamine ; de la 2'-méthoxy-3'-méthoxybiphényl-2,4-diamine ; de la 2'-méthoxy-3'-méthylbiphényl-2,4-diamine ; de la 2'-méthoxy-4'-aminobiphényl-2,4-diamine ; de la 2'-méthoxy-4'-hydroxybiphényl-2,4-diamine ; de la 2'-méthoxy-4'-méthoxybiphényl-2,4-diamine ; de la 2'-méthoxy-4'-méthylbiphényl-2,4-diamine ; de la 2'-méthoxy-5'-aminobiphényl-2,4-diamine ; de la 2'-méthoxy-5'-hydroxybiphényl-2,4-diamine ; de la 2'-méthoxy-5'-méthoxybiphényl-2,4-diamine ; de la 2'-méthoxy-5'-méthylbiphényl-2,4-diamine ; de la 2'-méthoxy-6'-aminobiphényl-2,4-diamine ; de la 2'-méthoxy-6'-hydroxybiphényl-2,4-diamine ; de la 2'-méthoxy-6'-méthoxybiphényl-2,4-diamine ; de la 2'-méthoxy-6'-méthylbiphényl-2,4-diamine ; de la 2'-méthyl-3'-aminobiphényl-2,4-diamine ; de la 2'-méthyl-3'-hydroxybiphényl-2,4-diamine ; de la 2'-méthyl-3'-méthoxybiphényl-2,4-diamine ; de la 2'-méthyl-3'-méthylbiphényl-2,4-diamine ; de la 2'-méthyl-4'-aminobiphényl-2,4-diamine ; de la 2'-méthyl-4'-hydroxybiphényl-2,4-diamine ; de la 2'-méthyl-4'-méthoxybiphényl-2,4-diamine ; de la 2'-méthyl-4'-méthylbiphényl-2,4-diamine ; de la 2'-méthyl-5'-aminobiphényl-2,4-diamine ; de la 2'-méthyl-5'-hydroxybiphényl-2,4-diamine ; de la 2'-méthyl-5'-méthoxybiphényl-2,4-diamine ; de la 2'-méthyl-5'-méthylbiphényl-2,4-diamine ; de la 2'-méthyl-6'-aminobiphényl-2,4-diamine ; de la 2'-méthyl-6'-hydroxybiphényl-2,4-diamine ; de la 2'-méthyl-6'-méthoxybiphényl-2,4-diamine ; de la 2'-méthyl-6'-méthylbiphényl-2,4-diamine ; de la 3'-amino-4'-hydroxybiphényl-2,4-diamine ; de la 3'-amino-4'-méthoxybiphényl-2,4-diamine ; de la 3'-amino-4'-méthylbiphényl-2,4-diamine ; de la 3'-amino-5'-aminobiphényl-2,4-diamine ; de la 3'-amino-5'-hydroxybiphényl-2,4-diamine ; de la 3'-amino-5'-méthoxybiphényl-2,4-diamine ; de la 3'-amino-5'-méthylbiphényl-2,4-diamine ; de la 3'-hydroxy-4'-aminobiphényl-2,4-diamine ; de la 3'-hydroxy-4'-hydroxybiphényl-2,4-diamine ; de la 3'-hydroxy-4'-méthoxybiphényl-2,4-diamine ; de la 3'-hydroxy-4'-méthylbiphényl-2,4-diamine ; de la 3'-hydroxy-5'-aminobiphényl-2,4-diamine ; de la 3'-hydroxy-5'-hydroxybiphényl-2,4-diamine ; de la 3'-hydroxy-5'-méthoxybiphényl-2,4-diamine ; de la 3'-hydroxy-5'-méthylbiphényl-2,4-diamine ; de la 3'-méthoxy-4'-aminobiphényl-2,4-diamine ; de la 3'-méthoxy-4'-hydroxybiphényl-2,4-diamine ; de la 3'-méthoxy-4'-méthoxybiphényl-2,4-diamine ; de la 3'-méthoxy-4'-méthylbiphényl-2,4-diamine ; de la 3'-méthoxy-5'-aminobiphényl-2,4-diamine ; de la 3'-méthoxy-5'-hydroxybiphényl-2,4-diamine ; de la 3'-méthoxy-5'-méthoxybiphényl-2,4-diamine ; de la 3'-méthoxy-5'-méthylbiphényl-2,4-diamine ; de la 3'-méthyl-4'-aminobiphényl-2,4-diamine ; de la 3'-méthyl-4'-hydroxybiphényl-2,4-diamine ; de la 3'-méthyl-4'-méthoxybiphényl-2,4-diamine ; de la 3'-méthyl-4'-méthylbiphényl-2,4-diamine ; de la 3'-méthyl-5'-aminobiphényl-2,4-diamine ; de la 3'-méthyl-5'-hydroxybiphényl-2,4-diamine ; de la 3'-méthyl-5'-méthoxybiphényl-2,4-diamine et de la 3'-méthyl-5'-méthylbiphényl-2,4-diamine, ainsi que de leurs sels physiologiquement acceptables.
